# EUROPEAN PATENT SPECIFICATION

(11) **EP 4 166 151 B1**
(45) Date of publication and mention of the grant of the patent: **18.02.2026**
(21) Application number: 20742306.2
(22) Date of filing: 10.06.2020
(51) Int. Cl.: A61K 35/747, A61P 15/08, G01N 33/50

(54) **PROBIOTIC FOR THE TREATMENT OF INFERTILITY AND RECURRENT PREGNANCY LOSS**
PROBIOTIKUM ZUR BEHANDLUNG VON UNFRUCHTBARKEIT UND REKURRENTEM SCHWANGERSCHAFTSVERLUST
PROBIOTIQUE POUR LE TRAITEMENT DE L'INFERTILITÉ ET DE LA PERTE DE GROSSESSE RÉCURRENTE

(43) Date of publication of application: 19.04.2023
(73) Proprietor: Biosearch S.A., 18004 Granada (ES)
(72) Inventor: FERNÁNDEZ ÁLVAREZ, Leónides, 28049 Madrid (ES); CASTRO NAVARRO, Irma, 28008 Madrid (ES); ARROYO RODRÍGUEZ, Rebeca, 28850 Torrejón de Ardoz, Madrid (ES); ALBA RUBIO, Claudio, 28400 Collado Villalba, Madrid (ES); RODRÍGUEZ GÓMEZ, Juan Miguel, 28770 Colmenar Viejo, Madrid (ES)
(74) Representative: Bird & Bird Società tra Avvocati S.r.l.
(86) International application number: PCT/ES2020/070382
(87) International publication number: WO 2021/250289

(56) References cited:
- EP-A1- 3 412 766
- MARTIN R ET AL: "Lactobacillus salivarius CECT 5713, a potential probiotic strain isolated from infant feces and breast milk of a mother-child pair", INTERNATIONAL JOURNAL OF FOOD MICROBIOLOGY, ELSEVIER BV, NL, vol. 112, no. 1, 15 October 2006 (2006-10-15), pages 35 - 43, XP024956312, ISSN: 0168-1605, [retrieved on 20061015], DOI: 10.1016/J.IJFOODMICRO.2006.06.011
- GUPTA PALLAVI ET AL: "Case Control Study to Compare Serum Vascular Endothelial Growth Factor (VEGF) Level in Women with Recurrent Pregnancy Loss (RPL) Compared to Women with Term Pregnancy", THE JOURNAL OF OBSTETRICS AND GYNECOLOGY OF INDIA, SPRINGER INDIA, INDIA, vol. 69, no. Suppl 2, 21 February 2018 (2018-02-21), pages 95 - 102, XP036911479, ISSN: 0971-9202, [retrieved on 20180221], DOI: 10.1007/S13224-018-1097-5
- MORENO INMACULADA ET AL: "Deciphering the effect of reproductive tract microbiota on human reproduction", REPRODUCTIVE MEDICINE AND BIOLOGY, vol. 18, no. 1, 1 November 2018 (2018-11-01), Tokyo, pages 40 - 50, XP055775954, ISSN: 1445-5781, Retrieved from the Internet <URL:https://api.wiley.com/onlinelibrary/tdm/v1/articles/10.1002%2Frmb2.12249> DOI: 10.1002/rmb2.12249
- BERNABEU ANDREA ET AL: "Effect of the vaginal microbiome on the pregnancy rate in women receiving assisted reproductive treatment", JOURNAL OF ASSISTED REPRODUCTION AND GENETICS, PLENUM PUBLISHING, US, vol. 36, no. 10, 24 August 2019 (2019-08-24), pages 2111 - 2119, XP037224706, ISSN: 1058-0468, [retrieved on 20190824], DOI: 10.1007/S10815-019-01564-0
- KOICHI KYONO ET AL: "Analysis of endometrial microbiota by 16S ribosomal RNA gene sequencing among infertile patients: a single-center pilot study", REPRODUCTIVE MEDICINE AND BIOLOGY, vol. 17, no. 3, 6 May 2018 (2018-05-06), Tokyo, pages 297 - 306, XP055694571, ISSN: 1445-5781, DOI: 10.1002/rmb2.12105

## Description

### FIELD OF THE INVENTION

The present invention belongs to the field of methods for the treatment of infertility and recurrent pregnancy loss.

### BACKGROUND OF THE INVENTION

Today, it is estimated that 15% of couples face fertility problems. In 33% of the cases, the woman is the source of the problem, 25% the man, 33% both the man and woman, but in 11% of the remaining cases the source is unknown.

There is mounting evidence showing the relevance of a woman's reproductive tract microbiota in human reproduction. Under physiological conditions, and in contrast to the intestinal microbiota, the human vaginal microbiota is generally characterized by a low bacterial diversity and the abundance and predominance of bacteria of the genus *Lactobacillus.*

The vaginal microbiota in most healthy women of reproductive age is mainly made up of one or several *Lactobacillus* species representing 90% to 95% of the total bacteria.

Several factors contribute to inter- and intra-individual changes in vaginal microbiota, and although changes may happen between the different vaginotypes, the increase in diversity and obligate anaerobes and the decrease in or depletion of lactobacilli are considered risk factors for bacterial vaginosis (BV).

The composition of the vaginal microbiota (and particularly any deviation of the Lactobacillus-dominated low-diversity vaginal microbiome) may play a key role in fertility and in the results of assisted reproductive treatments (ARTs). However, there are commercially available probiotics which are prescribed empirically to try to repopulate the female reproductive tract with *Lactobacillus* strains (Moreno and Simon, Reprod Med Biol. 2019; 18: 40-50) although no suitable scientific evaluation of their actual usefulness has been carried out.

There is therefore a need to provide new treatments for infertility, as well as for recurrent pregnancy loss, based on the administration of probiotics with proven efficacy.

### SUMMARY OF THE INVENTION

The authors of the present invention have discovered that, surprisingly, the oral administration of a probiotic containing *Lactobacillus salivarius* strain CECT 5713 to women with unexplained infertility or recurrent pregnancy loss allowed achieving pregnancy with an effectiveness of 66% (Table 4) and a reproductive success of 56% (Table 4). Moreover, the authors of the present invention have also discovered that, both in women with infertility and in women with recurrent pregnancy loss, treatment with the probiotic lead to a reduction in vaginal pH (Table 7) and an increase in vaginal TGFβ1, TGFβ2, and VEGF cytokine levels (Table 8) in those women who finally achieved pregnancy, but not in women who did not achieve pregnancy.

Therefore, in one aspect, the invention relates to a strain of the *Lactobacillus salivarius* species deposited in the Spanish Type Culture Collection (CECT) with accession number 5713 for use in the treatment and/or prevention of infertility or recurrent pregnancy loss in a female subject.

In another aspect, the invention relates to an *in vitro* monitoring method for monitoring the effect of a treatment for infertility or recurrent pregnancy loss in a female subject with a strain of the *Lactobacillus salivarius* species deposited in the CECT with accession number 5713 , which method comprises:
(i) determining the level of a growth factor selected from the group consisting of VEGF, TGF-β1, and TGF-β2 in a vaginal mucus sample and/or cervical mucus sample of said female subject, and
(ii) comparing said level with a reference value obtained from the same subject before therapy,
wherein an increase in said level with respect to the reference value is indicative of the treatment being effective, and
a decrease or a lack of change in said level with respect to the reference value is indicative of the treatment being ineffective.

### BRIEF DESCRIPTION OF THE DRAWINGS

**Figure 1****.** Dominant lactobacillus species in the cervicovaginal lavage samples of fertile women (C), women with repetitive miscarriage (RM), and women with infertility of unknown origin (INF), in those cases in which they could be isolated.
**Figure 2****.** Comparison of bacterial diversity evaluated by means of using the Shannon index (A) or the Simpson index (B) between the control, RM, and INF groups. The Wilcoxon rank-sum test with Bonferroni correction was used for pairwise comparisons between groups.
**Figure 3****.** PCoA (principal coordinate analysis) graphs of bacterial profiles (at the genus level) based on Bray-Curtis similarity analysis (relative abundance) (A) and Jaccard coefficient for binary data (presence/absence) (B) of the cervicovaginal lavage samples.
Control, circles; RM, crosses; INF, triangles. The box plots in the figures at the bottom show the distance of the samples to the centroid in each group. The value given on each axis represents the percentage of the total variance explained by said axis. The p-value was calculated with the permutation test for homogeneity of multivariate dispersions.

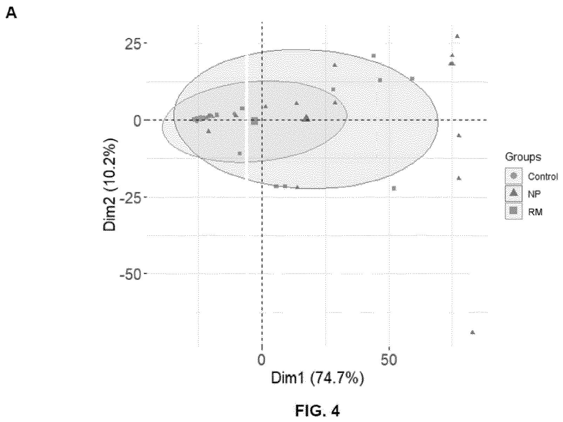

**Figure 4****.** PCA (principal component analysis) graphs of bacterial profiles at the genus level depending on their relative abundance in the cervicovaginal lavage samples (Control, circles; INF, triangles; RM, square).

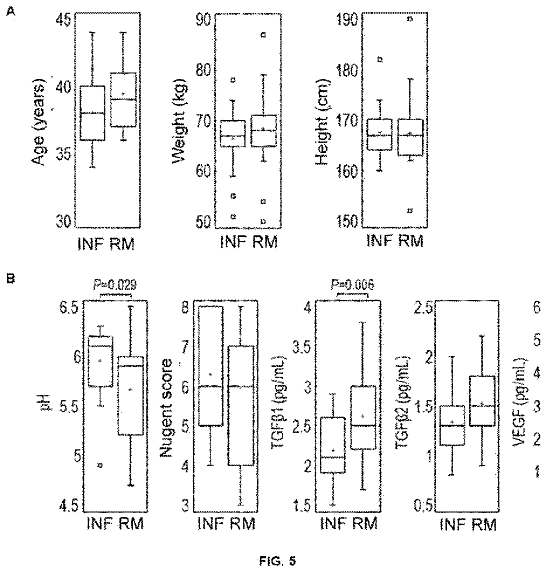

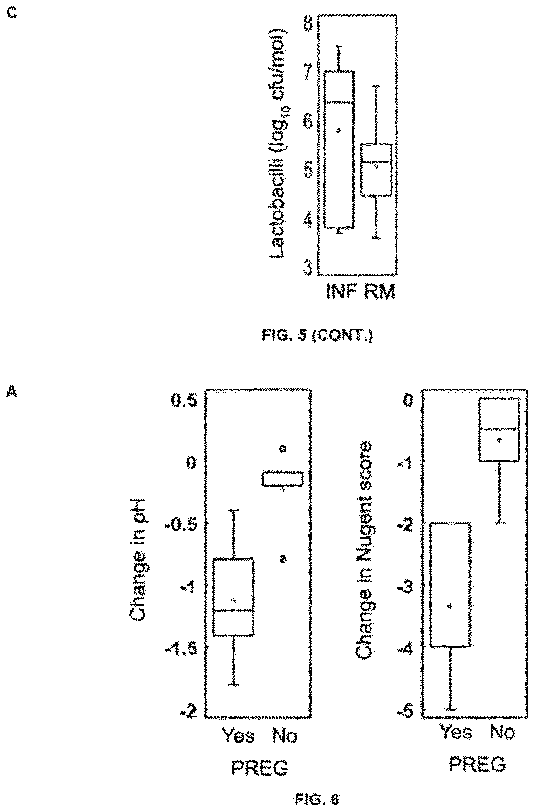

**Figure 5****.** Comparison of various characteristics between the RM group (women with a history of recurrent spontaneous miscarriage) and INF group (women with a history of infertility) at the time of recruitment: (A) age, weight, and height; (B) ph, Nugent score, and vaginal concentration of TGFβ1, TGFβ2, and VEGF, and (C) lactobacillus count.

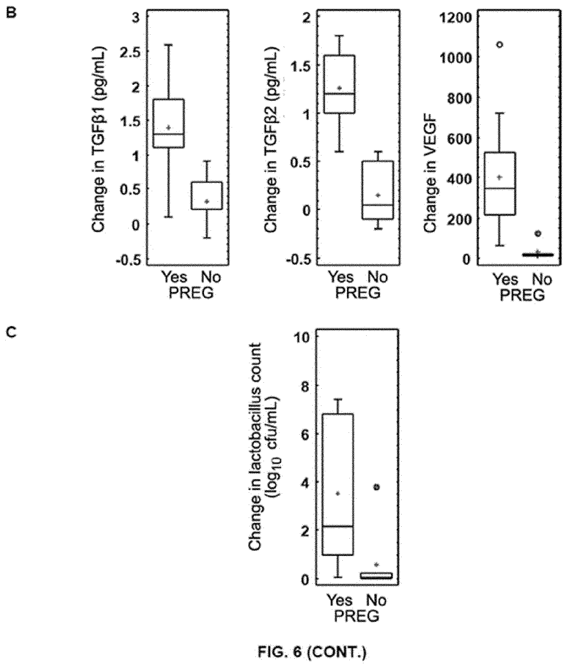

**Figure 6****.** Changes in characteristics of the RM group (women with a history of recurrent spontaneous miscarriage) after probiotic treatment, depending on the result (pregnant vs. not pregnant): (A) change in pH and Nugent score; (B) change in vaginal concentration of TGFβ1, TGFβ2, and VEGF; and (C) change in lactobacillus count.

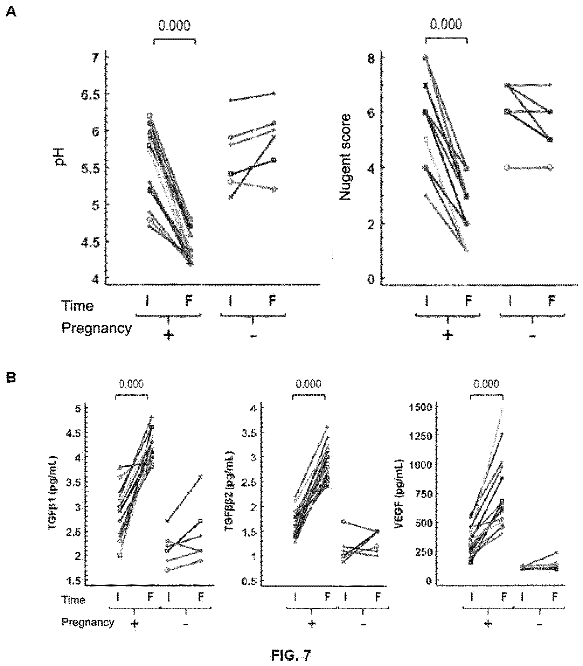

**Figure 7****.** Characteristics of the RM group (women with a history of recurrent spontaneous miscarriage) before (I) and after (F) probiotic treatment, depending on the result (pregnant vs. not pregnant). (A) pH and Nugent score; (B) vaginal concentration of TGFβ1, TGFβ2, and VEGF.

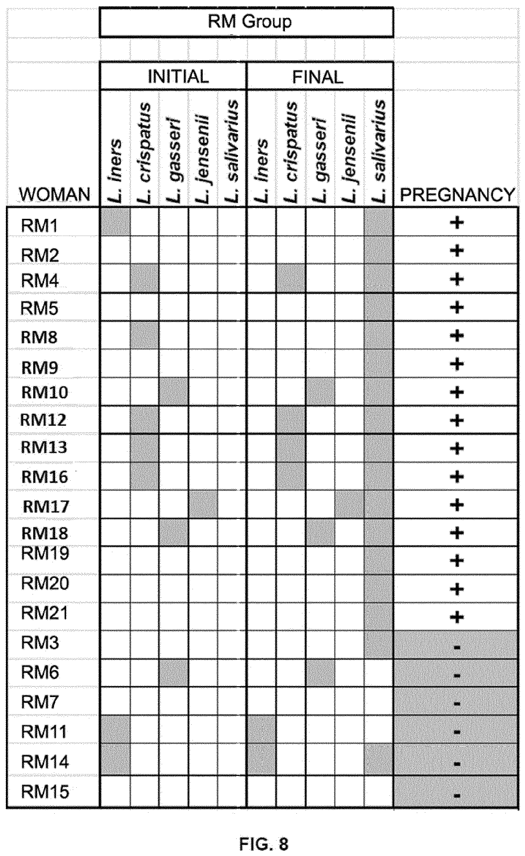

**Figure 8****.** Changes in the profile of dominant species of the genus *Lactobacillus* in the RM group (women with a history of recurrent spontaneous miscarriage) before (I) and after (F) probiotic treatment, depending on the result (pregnant vs. not pregnant).

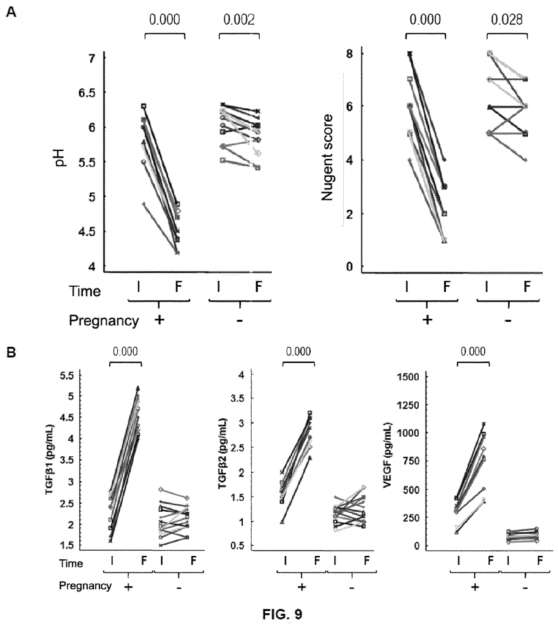

**Figure 9****.** Characteristics of the INF group (women with a history of infertility) before (I) and after (F) probiotic treatment, depending on the result (pregnant vs. not pregnant).

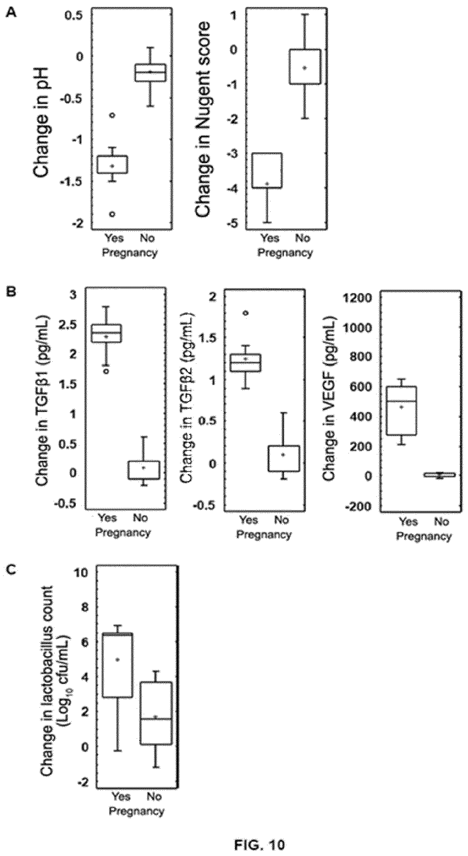

**Figure 10****.** Changes in characteristics of the INF group (women with a history of infertility) after probiotic treatment, depending on the result (pregnant vs. not pregnant): (A) change in pH and Nugent score; (B) change in vaginal concentration of TGFβ1, TGFβ2, and VEGF; and (C) change in lactobacillus count.

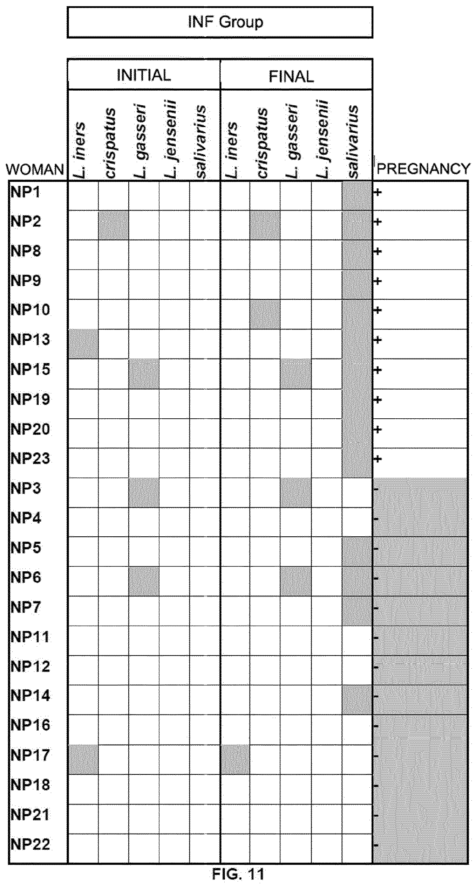

**Figure 11****.** Changes in the profile of dominant species of the genus *Lactobacillus* in the INF group (women with a history of infertility) before (I) and after (F) probiotic treatment, depending on the result (pregnant vs. not pregnant).

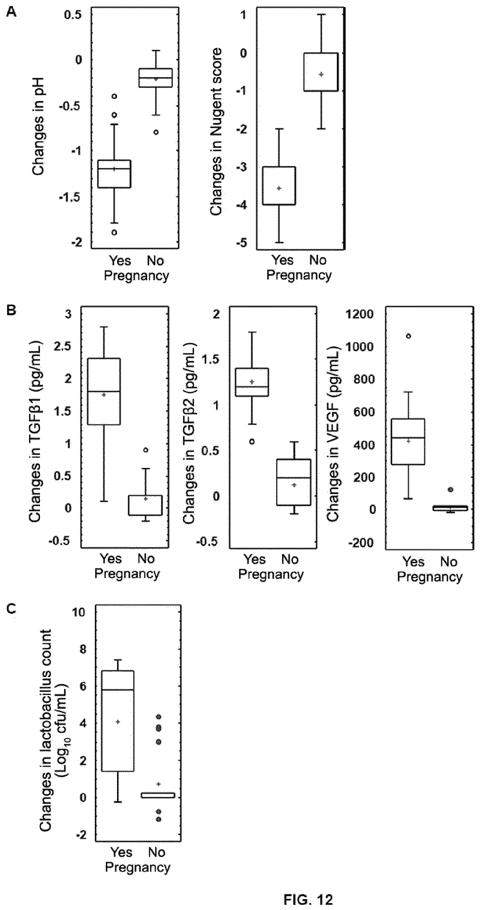

**Figure 12****.** Comparison of vaginal parameters among women in RM and INF groups (considered as a whole) who achieved pregnancy and those who did not: (A) change in pH and Nugent score; (B) change in vaginal concentration of TGFβ1, TGFβ2, and VEGF; and (C) change in lactobacillus count.

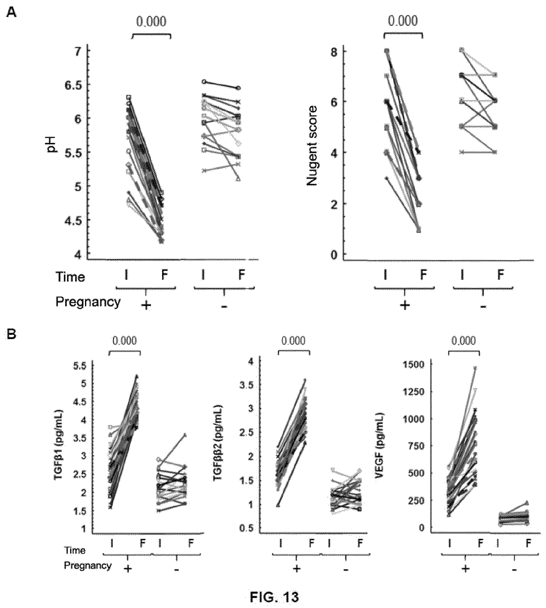

**Figure 13****.** Comparison of vaginal parameters among women in RM and INF groups (considered as a whole) who achieved pregnancy and those who did not before (I) and after (F) probiotic treatment, depending on the result (pregnant vs. not pregnant): (A) pH and Nugent score; (B) vaginal concentration of TGFβ1, TGFβ2, and VEGF.

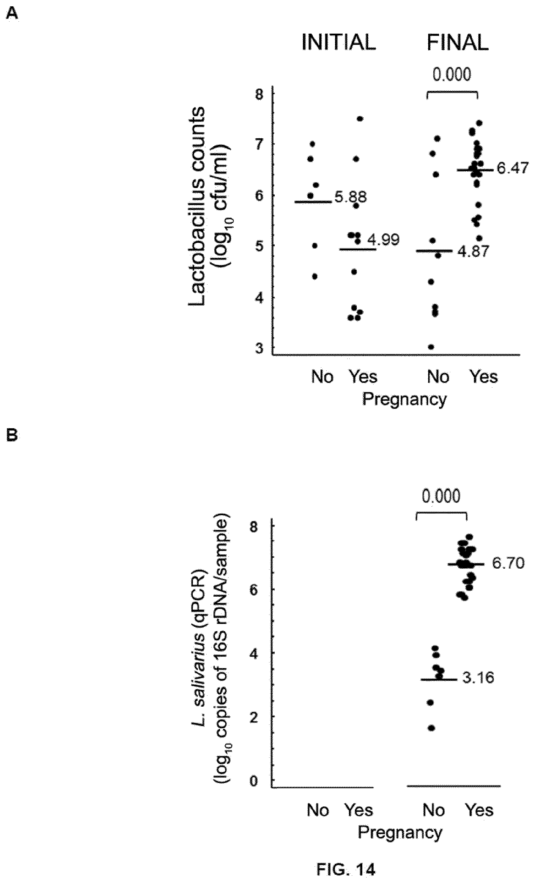

**Figure 14****.** Changes in lactobacillus counts (log₁₀ cfu/ml) (A) and in the number of *L*. *salivarius* specific DNA copies (copies/ml) (B) in cervicovaginal lavage samples of women of RM and INF groups (considered as a whole) who achieved pregnancy and those who did not before (I) and after (F) probiotic treatment, depending on the result (pregnant vs. not pregnant).

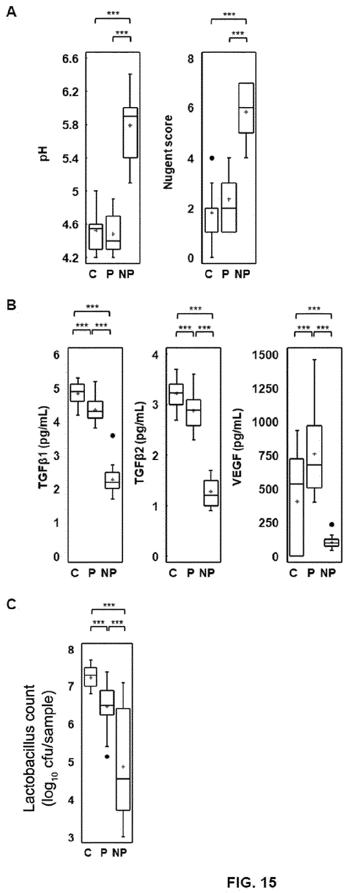

**Figure 15****.** Comparison of various characteristics of the control group with the characteristics of women in RM and INF groups who achieved pregnancy (P) and the characteristics of those who did not (NP) after probiotic treatment: (A) ph and Nugent score; (B) vaginal concentration of TGFβ1, TGFβ2, and VEGF; and (C) lactobacillus count.

### DETAILED DESCRIPTION OF THE INVENTION

### Medical uses of the invention

In one aspect, the invention relates to a strain of the *Lactobacillus salivarius* species deposited in the Spanish Type Culture Collection (CECT) with accession number 5713 for use in the treatment and/or prevention of infertility or recurrent pregnancy loss in a female subject.

Alternatively, the invention relates to the use of a strain of the *Lactobacillus salivarius* species deposited in the Spanish Type Culture Collection (CECT) with accession number 5713 for manufacturing a medicinal product for the treatment and/or prevention of infertility or recurrent pregnancy loss in a female subject.

Alternatively, the invention relates to a method for the treatment and/or prevention of infertility or recurrent pregnancy loss in a female subject which comprises administering to said subject a therapeutically effective amount of a strain of the *Lactobacillus salivarius* species deposited in the Spanish Type Culture Collection (CECT) with accession number 5713 .

As it is used herein, the term "strain" refers to a genetic variant or subtype of a species of microorganism, preferably a bacterial species.

The terms *"Lactobacillus salivarius"* and *"Lagilactobacillus salivarius"* are used as synonyms in the context of the present invention.

In one embodiment, the invention relates to a strain of the *Lactobacillus salivarius* species deposited in the Spanish Type Culture Collection (CECT) with accession number 5713 for the treatment and/or prevention of infertility or recurrent pregnancy loss in a female subject.

In another embodiment, the invention relates to a mutant of the strain of the *Lactobacillus salivarius* species deposited in the Spanish Type Culture Collection (CECT) with accession number 5713 for the treatment and/or prevention of infertility or recurrent pregnancy loss in a female subject.

As it is used herein, the term "mutant of the strain of the *Lactobacillus salivarius* species deposited in the Spanish Type Culture Collection (CECT) with accession number 5713" refers to any strain which is obtained naturally or developed specifically from the reference strain, mainly by mutation, and maintains the properties of the reference strain. *L. salivarius* strain 5713 is characterized by the presence of the following properties.
*(a) Antimicrobial activity against vaginal and*/*or cervical pathogens.*

The term "vaginal and/or cervical pathogen" refers to a microorganism, mainly a fungus or bacterium, capable of colonizing the mucosa of the vagina and/or cervix, potentially capable of causing a disease, such as vaginitis, vaginosis, trichomoniasis, candidiasis, or lower urinary tract infections, for example. The term "vaginal pathogen" includes both microorganisms of external origin and microorganisms which, while they may be present in a healthy vaginal mucosa, can give rise to a disease when an imbalance in the bacterial flora of the vagina occurs, including a decrease in certain microorganisms having a beneficial or protective effect and/or an increase in the potentially pathogenic microorganism. Non-limiting illustrative examples of vaginal pathogens include *Candida albicans, Candida glabrata, Candida tropicalis, Gardnerella vaginalis, Streptococcus agalactiae, Trichomonas vaginalis, Neisseria gonorrhoeae, Chlamydia trachomatis, Mycoplasma hominis, Prevotella, Peptostreptococcus, Escherichia coli, Enterococcus faecalis,* and *Ureaplasma urealyticum.* In a particular embodiment, the vaginal pathogen is selected from the group consisting of *Gardnerella vaginalis (G. vaginalis* MP14, *G. vaginalis* MP17, *G. vaginalis* MP20, *G. vaginalis* MP24, *G. vaginalis* MP29), *Streptococcus agalactiae (S. agalactiae* MP07, S. *agalactiae* MP12, S. *agalactiae* MP46), *Candida albicans (C. albicans* MP09, C. *albicans* MP18, C. *albicans* MP31), *Candida glabrata (C. glabrata* MP33, C. *glabrata* MP37), *Candida parapsilosis (C. parapsilosis* MP36, C. *parapsilosis* MP48), and *Ureaplasma urealyticum (U. urealyticum* MP39, *U. urealyticum* MP57).

As it is used herein, the term "antimicrobial activity" refers to the capacity to kill or inhibit the growth of a microorganism, in this case the pathogenic vaginal microorganism. Antimicrobial activity includes bactericidal activity (capacity to kill bacteria), bacteriostatic activity (capacity to inhibit bacterial growth), fungicidal activity (capacity of kill fungi), and fungistatic activity (capacity to inhibit fungal growth).

### (b) Capacity to co-aggregate with vaginal and cervical pathogens.

The term "vaginal and/or cervical pathogen" has been defined above. In a particular embodiment, the vaginal pathogen is selected from the group consisting of *Gardnerella vaginalis (G. vaginalis* MP14, *G. vaginalis* MP17, *G. vaginalis* MP20, *G*. *vaginalis* MP24, *G. vaginalis* MP29), *Streptococcus agalactiae (S. agalactiae* MP07, S. *agalactiae* MP12, S. *agalactiae* MP46), *Candida albicans (C. albicans* MP09, C. *albicans* MP18, C. *albicans* MP31), *Candida glabrata (C. glabrata* MP33, C. *glabrata* MP37), *Candida parapsilosis (C. parapsilosis* MP36, C. *parapsilosis* MP48), and *Ureaplasma urealyticum (U. urealyticum* MP39, *U. urealyticum* MP57). In a more particular embodiment, *L. salivarius* strain CECT 5713 has the capacity to co-aggregate with *G*. *vaginalis,* S. *agalactiae,* and C. *albicans.*

The term "capacity to co-aggregate" refers to the capacity of *L. salivarius* strain CECT 5713 to adhere to other microorganism of different species.

### (c) High adhesion to vaginal epithelial cells.

The term "adhesion" refers to the capacity of *L. salivarius* strain CECT 5713 to bind to the host tissues, in this case to vaginal epithelial cells. Adhesion to vaginal cells is considered high when it is at least 90% of the adhesion of *L. salivarius* CECT strain 9145 when determined under the same experimental conditions, more particularly at least 91%, at least 92%, at least 93%, at least 94%, at least 95%, at least 96%, at least 97%, at least 98%, at least 99%, 100%, or more.

### (d) High α-amylase activity

As it is used herein, the term "a-amylase activity" refers to the enzymatic activity identified by the number EC 3.2.1.1 capable of hydrolyzing glycosidic bonds along any point of the carbohydrate chain, breaking down the bonds to generate maltotriose and maltose from amylase and maltose, glucose and dextrin from amylopectin. The α-amylase activity of a bacterium is considered high when it is greater than 0.50 U/mL when determined by means of the method disclosed by Narita et al. (2006), Appl. Environ. Microbiol. 72: 269-275.

### (e) High lactic acid production.

As it is used herein, the term "lactic acid production" refers to the capacity of the strain to produce lactic acid, the official nomenclature of which is 2-hydroxypropanoic acid or α-hydroxy-propanoic acid, in any of its two L or D isomers. The lactic acid production of a bacterium is considered high when it is greater than 8 mg/ml in culture supernatants after incubation in optimal growth conditions.

As it is used herein, the term "treatment" refers to any type of therapy having the purpose of ending, improving, or reducing the susceptibility to suffer a clinical condition, as described herein. Therefore, "treatment," "treat," and their equivalent terms refer to obtaining a pharmacologically or physiologically desired effect, covering any treatment of a pathological condition or disorder in a mammal, including human beings. The effect can be prophylactic in terms of providing complete or partial prevention of a disorder and/or an adverse effect that can be attributed to same. In other words, "treatment" includes (1) inhibiting the disease, for example, stopping its development, (2) interrupting or ending the disorder or at least the symptoms associated therewith, so the patient would no longer suffer the disease or its symptoms, for example, causing the regression of the disease or its symptoms by means of restoring or repairing a lost, absent, or defective function, or stimulating an inefficient process, or (3) slowing down, alleviating, or improving the disease or the symptoms associated therewith, where slowing down is used in a broad sense to refer to at least a reduction in the magnitude of a parameter, such as inflammation, pain, or immune deficiency.

As it is used herein, the term "prevention," "preventing," or "prevent" refers to the administration of *L. salivarius* strain CECT 5713 to a female subject who has not been diagnosed with infertility or recurrent pregnancy loss at the time of administration, but who would normally be expected to develop said disease or to be at risk of said disease. Prevention seeks to hinder the onset of said disease. Prevention can be complete (for example, the complete absence of a disease). Prevention can also be partial, such that the onset of a disease in a subject is to a lesser extent than what would have occurred without the administration of the combination or composition of the present invention, for example. Prevention also refers to a reduced susceptibility to a clinical condition. Prevention also includes reducing the risk of suffering the disease.

The term "female subject" referred to herein applies to a member of the female sex of a mammalian animal species and includes but is not limited to domestic animals, primates, and humans; the subject is preferably a human being of any age or race.

*L. salivarius* strain CECT 5713 for use in the prevention and/or treatment according to the invention is administered in a therapeutically effective amount.

The term "effective" amount or "therapeutically effective amount" of a drug or pharmacologically active agent is understood to be a non-toxic amount of the drug or agent that is, however, sufficient to provide the desired effect. In the therapy of the present invention, an "effective amount" of *L. salivarius* strain CECT 5713 is the amount of said microorganism which is effective in providing the desired effect.

Although individual needs vary, the determination of the optimal ranges for the effective amounts of microorganism of the invention pertains to the common experience of those skilled in the art. In general, the dose required to provide an effective amount of said microorganism, which can be adjusted by one skilled in the art, will vary depending on age, health, physical condition, sex, diet, weight, treatment frequency, and the nature and degree of deterioration or disease, patient medical condition, the route of administration, pharmacological considerations such as the activity, efficacy, pharmacokinetic and toxicology profile of the particular compound used, if a system drug delivery is used, and if the compound is administered as part of a combination of drugs.

As it is used herein, the term "infertility" refers to the inability of a woman to reproduce by natural means. In particular, it is a disease of the reproductive system defined by the failure to achieve a clinical pregnancy after 12 months or more of regular unprotected sexual intercourse in the absence of another reason, such as breastfeeding or lactational amenorrhea. Female infertility can be due to different causes, including:
- Changes in menstrual cycle, including anovulation, hyperprolactinemia, polycystic ovary syndrome, luteal phase defect, premature ovarian failure.
- Diseases of the Fallopian tubes and uterus, including Fallopian tube obstruction, inflammation of the lesser pelvis (or upper genital infection), congenital uterine and vaginal malformations.
- Changes in cervical mucus
- Endometriosis
- Infections, such as untreated gonorrhea and chlamydia, chronic infections of the cervix, and the surgical treatment of cervical injuries associated with human papillomavirus (HPV) infection,
- Serious diseases such as cancer.
- Extreme thinness or obesity.
- Endocrine disorders, such as thyroid disease, hypothalamic dysfunction, and hyperprolactinemia.
- Autoimmune disorders such as lupus, Hashimoto's disease, and other types of thyroiditis, and rheumatoid arthritis.

In a particular embodiment, the infertility is unexplained infertility. As it is used herein, the term "unexplained infertility" or "idiopathic infertility" refers to infertility for which a cause cannot be identified after performing a series of diagnostic tests on both the woman and her male partner. In a particular embodiment, female infertility is considered unexplained infertility when:
- The physical examination does not show signs of anomalies in the uterus, Fallopian tubes, or ovaries.
- All ovarian reserve testing shows normal ovule supply levels.
- The hysterosalpingography (HSG) shows normal uterine cavity tubes and Fallopian tubes.
- The analysis of the semen obtained from the man shows suitable amounts of healthy spermatozoids.
- The ovulation activity of the woman is normal, or any ovulation disorder (such as polycystic ovary syndrome) has been successfully treated.
- All blood tests from both sexes show normal results.

As it is used herein, the term "recurrent pregnancy loss" or "recurrent spontaneous miscarriage" refers to a situation in which the female subject achieves a clinical pregnancy but said pregnancy does not reach its term. In particular, a recurrent pregnancy loss is considered to exist in the following cases:
- 3 consecutive pregnancy losses, including non-visualized pregnancy losses (European Society for Human Reproduction and Embryology, Royal College of Obstetricians and Gynaecologists)
- 2 or more pregnancy losses that are not necessarily consecutive, with the pregnancy having been documented by means of ultrasound or histopathological examination.
Recurrent pregnancy loss can be due to different causes, including:
- Anatomical causes: uterine malformations, fibroids.
- Lifestyle- or environment-related causes: tobacco use, drug abuse, excessive consumption of alcohol or caffeine, obesity.
- Untreated diseases such as thyroid disease or diabetes, abnormalities of the immune system or the blood coagulation system.

In a particular embodiment, the recurrent pregnancy loss is unexplained recurrent pregnancy loss. As it is used herein, the term "unexplained recurrent pregnancy loss" refers to a recurrent pregnancy loss for which a cause is not identified after performing a series of diagnostic tests on both the woman and her male partner. In a particular embodiment, the recurrent pregnancy loss is considered unexplained when the following tests are normal:
- Tests of the uterine cavity, aimed at detecting uterine abnormalities. These tests can include transvaginal ultrasound, hysterosalpingography, ultrasound with saline infusion, or magnetic resonance.
- Genetic testing on the woman and man, including testing the chromosomes of the tissue of a miscarried fetus and of the couple.
- Hormone testing including, among others, thyroid study and prolactin, follicle-stimulating hormone, fasting glucose, and insulin analyses.
- Hematological and immunological testing.

A female subject with infertility or recurrent pregnancy loss who will be subjected to an assisted reproductive treatment can be treated with *L. salivarius* strain CECT 5713 thereby increasing the chances of success of the assisted reproductive treatment. Therefore, in a particular embodiment, *L. salivarius* strain CECT 5713 is administered to a woman with infertility or recurrent pregnancy loss in combination with an assisted reproductive treatment.

As it is used herein, the term "assisted reproductive treatment" or "medically assisted reproductive treatment" refers to a medical treatment intended for favoring pregnancy in the case of infertility problems, whether male, female, or both male and female infertility problems, as well as in the case of women without a partner or with a female partner who wish to become pregnant with semen from a donor. Assisted reproduction is characterized by ovulation stimulation or induction and may comprise acting directly on gametes (oocytes and/or spermatozoids) to favor fertilization and the embryo transfer or deposition in the uterine cavity. Examples of assisted reproductive treatments include ovulation induction, controlled ovarian stimulation, scheduled intercourse, artificial intrauterine, intracervical, and intravaginal insemination, whether with semen from the partner or a donor, *in vitro* fertilization (IVF), sperm microinjection (ICSI), sperm retrieval, oocyte donation, fertility preservation.

In a particular embodiment, the administration of *L. salivarius* strain CECT 5713 or a mutant thereof in combination with an assisted reproductive treatment means that said strain is administered to a female subject who has received, is receiving, or will receive assisted reproductive treatment. In a particular embodiment, *L. salivarius* strain CECT 5713 is administered before treatment for a certain period of time, for example, for at least 1 month, at least 2 months, at least 3 months, at least 4 months, at least 5 months, at least 6 months, at least 7 months, at least 8 months, at least 9 months, at least 10 months, at least 11 months, at least 1 year before receiving assisted reproductive treatment. In a particular embodiment, *L. salivarius* strain CECT 5713 or a mutant thereof is administered throughout the entire period of time the female subject receives assisted reproductive treatment. In a particular embodiment, *L*. *salivarius* strain CECT 5713 is administered after the subject has received assisted reproductive treatment, for example, for at least 1 week, at least 2 weeks, at least 3 weeks, and in the event that pregnancy has been achieved, at least up to week 4, at least up to week 5, at least up to week 6, at least up to week 7, at least up to week 8, at least up to week 9, at least up to week 10, at least up to week 11, at least up to week 12, at least up to week 13, at least up to week 14, at least up to week 15 of pregnancy, or throughout the entire pregnancy. In a more particular embodiment, *L*. *salivarius* strain CECT 5713 or a mutant thereof is administered at least up to week 15 of pregnancy.

The term "week of pregnancy" refers to the time that elapsed from the last menstruation date, assuming that the menstrual cycle lasts for 28 days and that fertilization occurred on day 14 of said cycle.

*L. salivarius* strain CECT 5713 can be administered vaginally or orally, preferably orally.

In a particular embodiment, *L. salivarius* strain CECT 5713 is administered in the form of a lyophilisate.

The amount of *L. salivarius* strain CECT 5713 to be administered will vary depending on the subject and the particular mode of administration. Those skilled in the art will appreciate that the doses can also be determined with the guideline of Goodman and Goldman's The Pharmacological Basis of Therapeutics, 9th edition (1996), Appendix II, pages 1707-1711 and Goodman and Goldman's The Pharmacological Basis of Therapeutics, 10th edition (2001), Appendix II, pages 475-493. In a particular embodiment, *L. salivarius* strain CECT 57 is administered at a daily dose comprised between 5.0 log₁₀ cfu (colony forming units) and 13.0 log₁₀ cfu, for example, between 5.5 log₁₀ cfu and 12.5 log₁₀ cfu, between 6.0 log₁₀ cfu and 12.0 log₁₀ cfu, between 6.5 log₁₀ cfu and 11.5 log₁₀ cfu, between 7.0 log₁₀ cfu and 11.0 log₁₀ cfu, between 7.5 log₁₀ cfu and 10.5 log₁₀ cfu, between 8.0 log₁₀ cfu and 10.0 log₁₀ cfu, between 8.5 log₁₀ cfu and 9.5, about 9.0 log₁₀ cfu. Preferably, L. *salivarius* strain CECT 5713 or a mutant thereof is administered at a daily dose comprised between 8.5 log₁₀ cfu and 9.5 log₁₀ cfu, more preferably at a daily dose of about 9 log₁₀ cfu.

In a particular embodiment, *L. salivarius* strain CECT 5713 is administered for a period of time of at least 3 months, at least 4 months, at least 5 months, at least 6 months, at least 7 months, at least 8 months, at least 9 months, at least 10 months, at least 11 months, at least 1 year, preferably at least 3 months, or until the diagnosis of pregnancy, more preferably at least 6 months or until the diagnosis of pregnancy. If pregnancy is diagnosed, preferably *L. salivarius* strain CECT 5713 is administered at least up to week 4, at least up to week 5, at least up to week 6, at least up to week 7, at least up to week 8, at least up to week 9, at least up to week 10, at least up to week 11, at least up to week 12, at least up to week 13, at least up to week 14, at least up to week 15 of pregnancy, or throughout the entire pregnancy, more preferably at least up to week 15 of pregnancy.

### Monitoring method of the invention

The authors of the present invention have detected an increase in vaginal concentrations of growth factors VEGF, TGF-β1, and TGF-β2 induced by treatment with *L. salivarius* strain CECT 5713 in women who finally achieved pregnancy with respect to those who did not (Table 8), such that concentrations of these growth factors can be a treatment efficacy biomarker.

Therefore, in another aspect, the invention relates to an *in vitro* monitoring method for monitoring the effect of a treatment for infertility or recurrent pregnancy loss in a female subject with a strain of the *Lactobacillus salivarius* species deposited in the CECT with accession number 5713 , which method comprises:
(i) determining the level of a growth factor selected from the group consisting of VEGF, TGF-β1, and TGF-β2 in a vaginal mucus sample and/or cervical mucus sample of said female subject, and
(ii) comparing said level with a reference value obtained from the same subject before therapy,
wherein an increase in said level with respect to the reference value is indicative of the treatment being effective, and
a decrease or a lack of change in said level with respect to the reference value is indicative of the treatment being ineffective.

As it is used herein, the term *"in vitro"* refers to the method not being carried out on the body of a human or animal subject, but on a sample isolated from said subject.

The term "monitoring the effect of a treatment" refers to the response of the patient suffering a disease to the treatment for treating said disease.

The terms "infertility," "recurrent pregnancy loss," "female subject," "strain of the *Lactobacillus salivarius* species deposited in the CECT with accession number 5713," have been defined above in relation to the first inventive aspect. All the particular and preferred embodiments of the first aspect in relation to said terms also apply to the monitoring method for monitoring the treatment of the invention.

The monitoring method of the invention comprises determining the level of a growth factor selected from the group consisting of VEGF, TGF-β1, and TGF-β2 in a vaginal mucus sample and/or cervical mucus sample.

As it is used herein, the term "vaginal mucus sample and/or cervical mucus sample" refers to a sample from the surface of the vaginal and cervical mucosa and includes small molecules, extracellular polymers, proteins, and peptides, microbial cells, and host cells, in this case cells of the female subject. Said sample can be obtained by means of any known technique, for example, by means of swabbing or by means of washing with a fluid, for example, saline, which is known as "cervicovaginal" lavage or "CVL". In a particular embodiment, the vaginal mucus sample and/or cervical mucus sample is a cervicovaginal lavage.

As it is used herein, the term "level of VEGF, TGF-β1 or TGF-β2" refers to the amount of the corresponding proteins present in the sample. The level of a protein can be determined by means of any method known in the art suitable for determining and quantifying a protein in a sample. By way of non-limiting illustration, the level of a protein can be determined by means of a technique which comprises using antibodies with the capacity to bind specifically to the analyzed protein (or to fragments thereof containing the antigenic determinants) and then quantifying the resulting antigen-antibody complexes, or alternatively by means of a technique which does not comprise using antibodies such as, for example, by means of mass spectroscopy-based techniques. The antibodies can be monoclonal, polyclonal, or fragments thereof, Fv, Fab, Fab', and F(ab')2, scFv, diabodies, triabodies, tetrabodies, and humanized antibodies. Similarly, the antibodies can be labeled. Non-limiting illustrative examples of markers which can be used include radioactive isotopes, enzymes, fluorophores, chemiluminescent reagents, cofactors, or enzymatic substrates, enzymatic inhibitors, particles, or dyes. There is a wide range of known tests which can be used according to the present invention, such as the combined application of unlabeled antibodies (primary antibodies) and labeled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunoturbidimetry, immunofluorescence, techniques based on the use of biochips or protein microarrays including specific antibodies or assays based on colloidal precipitation in formats such as reactive strips and assays based on antibody-conjugated quantum dots. Other ways of detecting and quantifying proteins include, for example, affinity chromatography techniques or ligand-binding assays.

In a particular embodiment, the level of VEGF is determined by means of a multiplex immunoassay. In a particular embodiment, the level of TGF-β1 or TGF-β2 is determined by means of ELISA.

The term "VEGF," or "VEGF-A," or "vascular endothelial growth factor" refers to a member of the family of PDGF/VEGF growth factors. It is a heparin-binding protein that exists as a disulfide-linked homodimer. This growth factor induces vascular endothelial cell proliferation and migration and is essential for physiological and pathological angiogenesis. In humans, VEGF corresponds with the protein defined by accession number P15692 in the UniProtKB/Swiss-Prot database (version 248 of the entry, 22 April 2020; version 2 of the sequence, 16 November 2001).

The term "TGF-β1" or "transforming growth factor beta 1" refers to a protein belonging to the transforming growth factor beta superfamily of cytokines. It is a secretion protein involved in functions such as controlling cell growth, cell proliferation, differentiation processes, and apoptosis. In humans, TGF-β1 corresponds with the protein defined by accession number P01137 in the UniProtKB/Swiss-Prot database (version 253 of the entry, 22 April 2020; version 2 of the sequence, 1 February 1991).

The term "TGF-β2" or "transforming growth factor beta 2" refers to a protein belonging to the transforming growth factor beta superfamily of cytokines. It is a protein involved in functions such as angiogenesis and heart development. In humans, TGF-β2 corresponds with the protein defined by accession number P61812 in the UniProtKB/Swiss-Prot database (version 181 of the entry, 22 April 2020; version 1 of the sequence, 1 August 1988).

In one embodiment, the monitoring method of the invention comprises determining the level of VEGF in a vaginal mucus sample and/or cervical mucus sample. In one embodiment, the monitoring method of the invention comprises determining the level of TGF-β1 in a vaginal mucus sample and/or cervical mucus sample. In one embodiment, the monitoring method of the invention comprises determining the level of TGF-β2 in a vaginal mucus sample and/or cervical mucus sample.

In one embodiment, the monitoring method of the invention comprises determining the level of VEGF and TGF-β1 in a vaginal mucus sample and/or cervical mucus sample. In one embodiment, the monitoring method of the invention comprises determining the level of VEGF and TGF-β2 in a vaginal mucus sample and/or cervical mucus sample. In one embodiment, the monitoring method of the invention comprises determining the level of TGF-β1 and TGF-β2 in a vaginal mucus sample and/or cervical mucus sample.

In one embodiment, the monitoring method of the invention comprises determining the level of VEGF, TGF-β1, and TGF-β2 in a vaginal mucus sample and/or cervical mucus sample.

The monitoring method of the invention comprises comparing the level of VEGF, TGF-β1, and/or TGF-β2 with a reference value.

The level of VEGF, TGF-β1 and/or TGF-β2 is determined in a vaginal mucus sample and/or cervical mucus sample of the female subject taken after receiving treatment, specifically after receiving the administration of *L. salivarius* strain CECT 5713 for a period of time of at least 3 months, at least 4 months, at least 5 months, at least 6 months, at least 7 months, at least 8 months, at least 9 months, at least 10 months, at least 11 months, at least 1 year, preferably at least 3 months, or more preferably, at least 6 months.

As it is used herein, the term "reference value" refers to predetermined criteria used as a reference to evaluate the values or data obtained from the samples collected from a subject. The reference value can be an absolute value, a relative value, a value having an upper or lower limit, a range of values, a mean value, a median value, an average value, or a value compared with a particular control or baseline value. A reference value can be based on a value of an individual sample, such as a value obtained from the sample of the subject being analyzed, but at an earlier time, for example. In a particular embodiment, the reference value is the level of said growth factors in a vaginal mucus sample and/or cervical mucus sample before the female subject starts treatment with *L. salivarius* strain CECT 5713 for example, 4 weeks before, 3 weeks before, 2 weeks before, 1 week before, 6 days before, 5 days before, 4 days before, 3 days before, 2 days before, 1 day before, or the actual day said treatment begins.

According to the monitoring method of the invention, an increase in the level of any of VEGF, TGF-β1, and TGF-β2 with respect to its reference value is indicative of the treatment being effective, whereas a decrease or a lack of change in said level with respect to the reference value is indicative of the treatment being ineffective.

The term "increase in the level with respect to the reference value" indicates that the level of the growth factor is higher than its reference value. The level of the growth factors is considered to be higher than its reference value when it is higher by at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, at least 200% or higher with respect to the reference value.

The term "decrease or a lack of change in the level with respect to the reference value" indicates that the level of the growth factor is lower than or equal to its reference value. The level of the growth factor is considered to be lower than or equal to its reference value when it is lower than 1.5%, lower than 1.0%, lower than 0.5%, or equal to the reference value, when it is lower by at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100% with respect to the reference value.

In a particular embodiment, the infertility is unexplained infertility.

In a particular embodiment, the recurrent pregnancy loss is unexplained recurrent pregnancy loss.

In a particular embodiment, the treatment with *L. salivarius* strain CECT 5713 is administered in combination with an assisted reproductive treatment.

In a particular embodiment, the strain is administered orally.

In a particular embodiment, the strain is administered for a period of time of at least 3 months, at least 6 months, or until the diagnosis of pregnancy.

In a particular embodiment, if pregnancy is diagnosed, the administration of the strain is maintained at least until week 15 of pregnancy.

In a particular embodiment, the strain is administered at a daily dose comprised between 8.5 log₁₀ cfu and 9.5 log₁₀ cfu. In a more particular embodiment, the strain is administered at a daily dose of about 9 log₁₀ cfu.

The invention is described below by means of the following merely illustrative examples that do not limit the scope of the invention.

### EXAMPLES

### Materials and methods

### Participants, sample, and design of the study

A total of 58 women between 28 and 45 years of age participated in this study. The volunteers were distributed in 3 groups. All the volunteers in the RM group (n = 21) had histories of recurrent spontaneous miscarriage with three or more pregnancy losses during the first 12 weeks of pregnancy. All the women in the INF group (n = 23) had a history of infertility (inability to conceive) despite having received ART for at least three times, including at least one *in vitro* fertilization (IVF) cycle. Finally, the control group (n = 14) included control women having at least two children after full-term pregnancy without complications. None of the women in the RM and INF groups received ART for the entire period of the study. None of the women in the RM group was diagnosed with antiphospholipid syndrome, and therefore did not receive heparin and/or salicylic acid during the study. None of the women participants had received or taken hormone therapy, antibiotics, or probiotics 4 weeks prior to sampling. Vaginal samples were taken at least 7 days after intercourse to prevent or minimize the impact of the partner's semen on the vaginal pH, microbiome, and immunological profile (in the last case, particularly in relation to the concentration of TGF-β1 and TGF-β2). Women with lactose intolerance or allergy to cow's milk protein were excluded due to the excipient used for administering the strain in the subsequent pilot test (see below).

During recruitment (day 0), two samples were collected from women in the three groups within the first three days after ovulation: a vaginal swab sample for determining the Nugent score again and a cervicovaginal lavage (CVL) of the cervical opening and vaginal walls with 10 ml of sterile normal saline (Nakra et al., J Infect Dis. 2016, 213(5): 840-7) for all other analyses. Aliquot parts of the CVL samples were used for culture-based analysis. The CVL samples were then clarified by low-speed centrifugation at 800 × g for 10 minutes at 4°C. CVL supernatant aliquots and cell pellets were stored at -80°C until immunological and metataxonomic analyses were performed.

After day 0, women in the RM and INF groups (orally) took a daily sachet with ~ 50 mg of lyophilized probiotic (~ 9 log₁₀ cfu of *L. salivarius* CECT 5713) for 6 months or until the diagnosis of pregnancy (whichever occurred first). At this point, the same two samples were collected from each woman. After a diagnosis of pregnancy, the oral administration of the strain was maintained until week 15 of pregnancy. All spontaneous pregnancies occurring within the first year after day 0 were recorded in this study.

The sachets containing the probiotic were kept at 4°C during the entire study. All the volunteers received questionnaires to record adherence with the intake of the products of the study. The minimum rate of adherence (% of total treatment doses) was established at 86%. All the volunteers gave their written informed consent for the protocol which has been approved by the Biomedical Research Ethics Committee of the Health and Family Department (Regional Government of Andalusia, Granada, Spain) (P050/19, Law 11/19).

### Measurement of vaginal pH and Nugent score

The pH of the lateral vaginal wall (Whatman pH paper, pH 3.8-5.5 and pH 6.0-8.1) was measured in each of the two study visits. The Nugent score was computed as described previously (Nugent et al., J Clin Microbiol. 1991, 29(2): 297-301). Briefly, the material of the swab was transferred to a glass slide, heat fixed, and Gram stained. The Gram positive, Gram negative, and Gram variable bacterial morphotypes were quantified. A Nugent score of 0-3 was considered normal, 4-6 intermediate, and 7-10 compatible with bacterial vaginosis (Nugent *et al., supra).*

### Culture-dependent analysis

The CVL samples collected during the assay were serially diluted and placed on plates with Columbia nalidixic acid (CNA) agar, Gardnerella (GAR) agar, CHROMagarStrepB (CHR) agar, MacConkey (MCK) agar, Mycoplasma (MYC) agar, and Sabouraud Dextrose Chloramphenicol (SDC) agar (BioMerieux, Marcy L'Etoile, France) for the selective isolation and quantification of the main cultivable non-*Lactobacillus* bacteria and yeasts which can be found in the vagina, including the agents most frequently involved in vaginal infections. The samples were also spread over plates with MRS agar (Oxoid, Basingstoke, United Kingdom) supplemented with L-cysteine (2.5 g/I) (MRS-C) or horse blood (5%) (MRS-B) for the isolation of lactobacilli, including *L*. *iners* (MRS-B). All the plates were incubated for 48 hours at 37°C under aerobic conditions, with the exception of MRS-C and MRS-B, which were incubated anaerobically (85% nitrogen, 10% hydrogen, 5% carbon dioxide) in an anaerobic work station (DW Scientific, Shipley, United Kingdom). After incubation, the counts in each growth medium were recorded and at least one representative of each morphology of the colony was then selected from the agar plates. The isolates were identified by means of matrix-assisted laser desorption/ionization-time of flight (MALDI-TOF) mass spectrometry (Bruker, Germany). When identification by means of MALDI-TOF was not possible at the species level (particularly in the case of lactobacillus isolates), identification was carried out by means of sequencing the 16S ribosomal RNA (rRNA) gene as described by Mediano et al. (J Hum Lact. 2017, 33(2): 309-318)

### Extraction of DNA from the samples

About 1 ml of each CVL sample was used for DNA extraction following a previously described method (Lackey et al., Front Nutr. 2019, 17; 6:45). The extracted DNA was eluted in 22 µl of nuclease-free water and stored at -20°C until subsequent analysis. The purity and concentration of each extracted DNA were estimated using a NanoDrop 1000 spectrophotometer (NanoDrop Technologies, Inc., Rockland, United States). Negative controls (without sample) were added during extraction.

### Detection and quantification of Lactobacillus salivarius DNA by means of qPCR (real-time quantitative PCR)

The primers and conditions for quantifying the DNA belonging specifically to the species *L. salivarius* have been described previously (Harrow et al., Appl Environ Microbiol. 2007, 73(22): 7123-7). The DNA concentration of all the samples was adjusted to 5 ng µL⁻¹. A commercial real-time PCR thermocycler (CFX96^{™}, Biorad Laboratories, Hercules, CA, United States) was used for all the experiments. Standard curves using 1:10 dilutions (varying from 2 ng to 0.2 pg) of *L. salivarius* CECT 5713 DNA were used for calculating the unknown bacterial genomic targets. Threshold cycle (Ct) values between 15.29 and 20.07 were obtained for this range of *L. salivarius* DNA (R2 = 0.9915). The Ct values measured for the DNA extracted from non-target species (L. *reuteri* MP07 and *L. plantarum* MP02; collection belonging to applicant) were ≥39.27 ± 0.64. These two control strains were selected because they belong to the species that is taxonomically closest to *L. salivarius* (Salvetti *et al.,* 2018). All the samples and standards were processed in triplicate.

### Metataxonomic analysis

The hypervariable regions V3-V4 of 16S rDNA were amplified by means of PCR using universal primers SD-Bact-0341-bS-17 (CCTACGGGNGGCWGCAG, SEQ ID NO: 1) and SD-Bact-129 0785-aA-21 (GACTACHVGGGTATCTAATCC, SEQ ID NO: 2) and sequenced in the Illumina MiSeq system in the Madrid Science Park facilities (Tres Cantos, Spain) (Klindworth et al., Nucleic Acids Res. 2013, 41, e1). The barcodes appended to the 3' and 5' terminal ends of the PCR amplicons in a second PCR reaction allowed separating forward and reverse sequences. The DNA concentration in the PCR products was quantified through the 2100 Bioanalyzer system (Agilent, Santa Clara, CA, United States). After grouping the PCR products at about the same molar proportions, the DNA amplicons were purified using a QIAEX II gel extraction kit (Qiagen) on the removed band which had the correct size after running on agarose gel. The DNA concentration was then quantified with PicoGreen (BMG Labtech, Jena, Germany). The barcode-bearing grouped and purified DNA amplicons were sequenced using the Illumina MiSeq paired-end protocol (Illumina Inc., San Diego, CA, United States) following the manufacturer's protocols.

### Analysis of immunological parameters

The vascular endothelial growth factor (VEGF) was analyzed using a premixed multiplex panel (Bio-Rad, San Diego, CA). The levels of TGF-β 1 and TGF-β 2 were measured in parallel by means of ELISA using RayBio^{®} Human TGF-β1 and Human TGF-β2 ELISA kits, respectively (RayBiotech, Norcross, GA, United States). All the determinations were carried out following the manufacturer's protocol and standard curves were plotted for each analyte.

### Antimicrobial activity of L. salivarius CECT 5713 against vaginal pathogens

A superposition method (Magnusson and Schnürer, Appl. Environ. Microbiol. 2001, 67, 1-5) was used for determining the capacity of *L. salivarius* CECT 5713 to inhibit the growth of various bacterial and yeast species. This method was performed as described by Martin *et al.* (2006). All the strains used as indicator organisms (culture collection belonging to the applicant) had been previously isolated from clinical cases of vaginal or cervical infections, and included five strains of *Gardnerella vaginalis,* three strains of *Streptococcus agalactiae,* three strains of *Candida albicans,* two strains of *Candida glabrata,* two strains of *Candida parapsilosis,* and two strains of *Ureaplasma urealyticum.* The inhibitory activity was tested in triplicate in all the experiments.

### Production of antimicrobial compounds by L. salivarius CECT 5713

The production of hydrogen peroxide by the *Lactobacillus* strain was measured using the quantitative method of Yap and Gilliland, 2000, J. Dairy Sci. 83, 628-632, modified by Martin et al. (Int. J. Food Microbiol. 2006, 112: 35-43). The production of lactic acid (L and D isomers) and acetic acid by *L. salivarius* CECT 5713 was determined using enzymatic kits (Roche Diagnostics, Mannheim, Germany) as described by Martin *et al.* (2006). The pH values of the culture supernatants were also measured. These assays were performed in triplicate under strict aerobic and anaerobic conditions, and the values were expressed as mean ± SD. The bacteriocin activity was not analyzed because earlier phenotypic analyses and analysis of the *L. salivarius* CECT 5713 genome have demonstrated that this strain is incapable of producing such antimicrobial compounds (Martin *et al.,* supra; Langa et al., Appl Microbiol Biotechnol. 2012, 94(5):1279-87).

### Co-aggregation and vaginal epithelial cell line adhesion assays

The capacity of the strain to form an aggregate with cells of the aforementioned indicator strains was investigated following the method of Younes et al. (PLoS One 2012, 7, e36917). The suspensions were observed under a phase-contrast microscope after Gram staining. Adhesion to vaginal epithelial cells collected from healthy premenopausal women was carried out and interpreted as described previously (Boris et al., Infect. Immun. 1998, 66, 1985-1989). Adhesion was measured as the number of lactobacilli adhered to the vaginal cells in 20 random microscopic fields. *L. salivarius* CECT 9145 was used as the control strain due to its high adhesion to vaginal cells (Martin et al., Nutrients. 2019, 11(4). pii: E810). The assay was performed in triplicate.

### α-Amylase activity of L. salivarius CECT 5713

On one hand, the α-amylase activity of *L. salivarius* CECT 5713 was evaluated qualitatively using the method described by Padmavathi et al. (J Genet Eng Biotechnol. 2018; 16(2):357-362). Briefly, the strain was inoculated in a modified MRS medium (0.5% peptone, 0.7% yeast extract, 0.2% NaCl, 2% starch, and 1.5% agar) supplemented with 0.25% starch. The plates were incubated at 37°C for 48 h in anaerobiosis and the zone of clearance was then observed by adding Gram's iodine as the detection agent. Next, the α-amylase activity on the surface of *L. salivarius* CECT 5713 was also measured with an α-amylase measurement kit (Kikkoman Co., Tokyo, Japan) using 2-chloro-4-nitrophenyl 6⁵-azido-6⁵-deoxy-β-maltopentaosideas substrate using a protocol and conditions described previously (Narita *et al.,* 2006). One activity unit was defined as the amount of enzyme required for releasing 1 µmol of 2-chloro-4-nitrophenol from 2-chloro-4-nitrophenyl 6⁵-azido-6⁵-deoxy-β-maltopentaoside per minute at 37°C.

### Bioinformatics analysis

The unprocessed raw data was demultiplexed and quality-filtered using the Illumina MiSeq Reporter analysis software. The microbiome bioinformatics analysis was performed with QIIME 2 2019.1 (Bolyen et al., Nature Biotechnology, 2019, 37: 852-857). Disinfection was performed with DADA2 (Callahan et al., Nat Methods. 2016; 13: 581-583). Taxonomy was assigned to ASVs using the q2 feature classifier (Bokulich et al. Microbiome. 2018, a; 6:90) which classifies the Sklearn naive Bayes taxonomy classifier against the SILVA 132 OTU reference sequences (Quast, Nucl. Acids Res. 2013, 41 (D1): D590-D596). The subsequent bioinformatics analysis was performed using version R 3.5.1 (R Core Team, 2013; https://www.R-project.org). A table of OTU counts per sample was generated, and the abundances of bacterial taxons were normalized with the total number of sequences in each sample. Alpha and diversity were studied with Shannon and Simpson diversity indices with the R Vegan package (Version: 2.5.6) (Oksanen et al., Community Ecology package, 2007, 10; 631-637). Beta diversity studies were performed using principal coordinate analysis (PCoA) for plotting the bacterial community diversity patterns through a distance matrix containing a dissimilarity value for each paired sample comparison. Quantitative (relative abundance) and qualitative (presence/absence) analyses were performed with the Bray-Curtis index and Jaccard binary index, respectively. The analysis of variance of the distance matrices was performed with Adonis "non-parametric MANOVA test" with 999 permutations to reveal statistical significance with the R Vegan package.

### Statistical analysis

The microbiological data was recorded as CFU/ml and transformed to log values before statistical analysis. Quantitative data was expressed as the median and the interquartile range (IQR). Differences between groups were evaluated by means of Kruskal-Wallis tests and paired Wilcoxon rank-sum tests for calculating comparisons between groups. The Bonferroni correction test was performed. The correlation between the 20 relatively abundant genera was viewed using the R "qgraph" package (Epskamp 2012). Statistical analysis and plotting were performed in the R environment with the ggplot2 library (Wickham, 2016). Differences with a p-value <0.05 were considered statistically significant. The contribution of a variable is (in percentage form): (cos2 variable * 100) / (total cos2 of the component). Cos2 = quality of representation or weight of the variable in the PCA representation.

### Results

### Demographic data and clinical characteristics of the participants

The characteristics of the 58 women who participated in this study are shown in Table 1. The mean age (CI 95%) of the women in the control group was 34.6 years (33.4-35.8 years), whereas the mean age of women with repetitive miscarriages (RM) and women with infertility of unknown origin (INF) was 39.4 (38.5-40.4) years and 38.0 (37.1-38.9) years (Table 1). The women in the control group were about 4-5 years younger than other participants (p <0.001; one-way ANOVA), but there were no differences in the mean values of body weight and height between the three groups of women (Table 1).

**Table 1. Characteristics of the participants (n = 58) who were distributed into three groups: control group (women having at least two children after full-term pregnancy without complications), RM group (women with a history of recurrent miscarriage), and INF group (women with a history of infertility).**

| | | **Control** | **RM** | **INF** | |
|---|---|---|---|---|---|
| | | ***n=*14** | ***n=*21** | ***n=*23** | ***p*-value#** |
| **Age (years)** | | | | | |
| | Mean | 34.6 | 39.4 | 38.0 | <0.001 |
| | SD | 4.5 | 2.4 | 2.7 | |
| | CI 95% | (33.5-35.8) | (38.5-40.4) | (37.1-38.9) | |
| | [min-max] | [280-450] | [36.0-44.0] | [34.0-44.0] | |

| **Weight (kg)** | | | | | |
|---|---|---|---|---|---|
| | Mean | 62.4 | 68.3 | 66.5 | 0.054 |
| | SD | 6.7 | 8.1 | 6.0 | |
| | CI 95% | (59.7-65.0) | (66.1-70.4) | (64.5-68.6) | |
| | [min-max] | [46.0-87.0] | [50.0-87.0] | [51.0-78.0] | |

| **Height (cm)** | | | | | |
|---|---|---|---|---|---|
| | Mean | 166 | 167 | 168 | 0.761 |
| | SD | 5.64 | 7.47 | 5.08 | |
| | CI 95% | (164-168) | (165-169) | (166-169) | |
| | [min-max] | [156-175] | [152-190] | [160-182] | |

| **Menstrual cycle** | | | | | |
|---|---|---|---|---|---|
| | **Regular [n (%)]** | | | | |
| | Yes | 10 (71) | 11 (48) | 10 (48) | 0.337* |
| | No | 4 (29) | 12 (52) | 11 (52) | |

| **Duration (days)** | | | | | |
|---|---|---|---|---|---|
| | Mean | 28.0 | 27.4 | 27.5 | 0.502 |
| | SD | 2.3 | 1.4 | 1.6 | |
| | CI 95% | (27.4-28.7) | (26.9-27.9) | 27.0-28.0) | |
| | [min-max] | [250-325] | [24.0-30.0] | [24.0-30.0] | |

| | | | | | |
|---|---|---|---|---|---|
| SD: standard deviation; Cl: confidence interval. # One-way ANOVA tests were used to evaluate the differences in mean age, mean weight, mean height, and mean duration of menstrual cycle between groups. *Freeman-Halton extension of Fisher exact probability tests for a 2 × 3 contingency table. | | | | | |

About 71% of women in the control group had a regular menstrual cycle, whereas in the other two groups (RM, and INF) this percentage was 48%, although this difference was not statistically significant (p = 0.337; Freeman-Halton extension of Fisher exact probability tests for a 2 × 3 contingency table). No differences were observed in the mean duration of menstrual cycle which was 28, 27.4, and 27.5 days for the women in the control, RM, and INF groups, respectively (Table 1).

### Baseline vaginal health parameters

Women in the control group had a mean vaginal pH (Cl 95%) of 4.53 (4.38-4.68) (Table 2). In contrast, vaginal pH was higher in the two groups being studied: 5.67 (5.55-5.79) in the RM group and 5.96 (5.84-6.07) in the INF group (p = 0.000; one-way ANOVA). Mean Nugent scores with higher significance (Cl 95%), 5.95 (5.54-6.37) and 6.30 (5.91-6.70) were recorded in women from the RM and INF groups, respectively, in comparison with the controls (p = 0.000; one-way ANOVA) (Table 2). Differences were also observed in the levels of cytokines TGFβ1, TFGβ2, and VEGF in CVL. The CVL content of these three cytokines in women from the control group was 4.83 pg/ml (4.65-5.01) pg/ml, 3.22 pg/ml (4.65-5.01) pg/ml, and 406.0 pg/ml (322.0-490.0) pg/mL for TGFβ1, TFGβ2, and VEGF, respectively, whereas they were reduced to about half in both groups being studied (RM and INF) (Table 2).

**Table 2. Comparison of baseline vaginal parameters (pH, Nugent score, cytokines, and microbiology) of the participants (n = 58) from the control group (women having at least two children after full-term pregnancy without complications), RM group (women with a history of recurrent miscarriage), and INF group (women with a history of infertility).**

| | | **Control** | **RM** | **INF** | ***p-*value#** |
|---|---|---|---|---|---|
| | | ***n=*14** | ***n=*21** | ***n=*23** | |
| **pH** | | | | | |
| | Mean | 4.53**a** | 5.67**b** | 5.96**c** | 0.000 |
| | SD | 0.25 | 0.50 | 0.34 | |
| | CI 95% | (4.38-4.68) | (5.55-5.79) | (5.84-6.07) | |
| | [min-max] | [4.20-5.00] | [4.70-6.50] | [4.90-6.30] | |

| **Nugent score** | | | | | |
|---|---|---|---|---|---|
| | Mean | 1.79**a** | 5.95**b** | 6.30**b** | 0.000 |
| | SD | 0.98 | 1.60 | 1.29 | |
| | CI 95% | (1.27-2.30) | (5.54-6.37) | (5.91-6.70) | |
| | [min-max] | [0.00-4.00] | [3.00-8.00] | [4.00-8.00] | |

| **TGFβ1 (pg/mL)** | | | | | |
|---|---|---|---|---|---|
| | Mean | 4.83**a** | 2.62 **b** | 2.19**c** | 0.000 |
| | SD | 0.36 | 0.57 | 0.42 | |
| | CI 95% | (4.65-5.01) | (2.47-2.76) | (2.05-2.33) | |
| | [min-max] | [4.20-5.30] | [1.70-3.80] | [1.50-2.90] | |

| **TGFβ2 (pg/mL)** | | | | | |
|---|---|---|---|---|---|
| | Mean | 3.22**a** | 1.52**b** | 1.33**b** | 0.000 |
| | SD | 0.27 | 0.36 | 0.30 | |
| | CI 95% | (4.65-5.01) | (2.47-2.76) | (2.05-2.33) | |
| | [min-max] | [2.70-3.70] | [0.90-2.20] | [0.80-2.00] | |

| **VEGF (pg/mL)** | | | | | |
|---|---|---|---|---|---|
| | Mean | 406.0**a** | 275.0**ab** | 181.0**b** | 0.016 |
| | SD | 379.0 | 150.0 | 134.0 | |
| | CI 95% | (322.0-490.0) | (206.0-343.0) | (116.0-247.0) | |
| | [min-max] | [1.4-929.0] | [95.0-562.0] | [38.0-431.0] | |

| **Lactobacilli** | | | | | |
|---|---|---|---|---|---|
| | Positive women [n, (%)] | 14 (100) | 12 (57) | 6 (26) | <0.001* |
| | Count (log₁₀cfu/swab) | | | | |
| | Mean** | 7.24**a** | 5.04**b** | 5.78**b** | 0.000 |
| | SD | 0.30 | 0.94 | 1.65 | |
| | CI 95% | (6.89-7.60) | (4.66-5.42) | (5.24-6.32) | |
| | [min-max] | [6.80-7.70] | [3.60-6.70] | [3.70-7.50] | |

| | | | | | |
|---|---|---|---|---|---|
| SD: standard deviation; Cl: confidence interval, TGFβ1, transforming growth factor β1; TGFβ2, transforming growth factor β2; VEGF, vascular endothelial growth factor. # One-way ANOVA tests were used to evaluate the differences in the means between groups. The different letters shown in bold in the same row indicate statistically significant differences between groups. *Freeman-Halton extension of Fisher exact probability tests for a 2 × 3 contingency table. **Mean in those women in whom lactobacilli could be isolated from their samples. | | | | | |

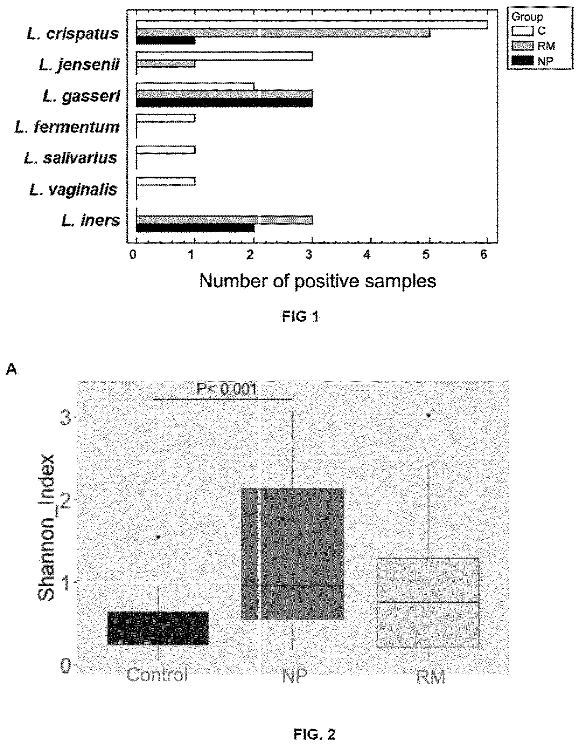

Lactobacilli were detected in the CVL of all the women in the control group (n = 14) and the mean value (Cl 95%) of the lactobacillus counts was 7.24 log cfu/ml (6.89-7.60 log10 cfu/ml) using a culture-dependent evaluation. The CVL lactobacillus detection frequency was lower in the RM and INF groups: 57% and 26%, respectively (p <0.001, Freeman-Halton extension of Fisher exact probability tests for a 2 × 3 contingency table). Furthermore, the lactobacillus concentrations in the CVL samples of lactobacillus-positive women were also 2.20 and 1.46 log10 units lower in RM and INF groups, respectively. Another difference between these groups was in relation to the lactobacillus profile (Figure 1). Up to six different lactobacillus species were identified in the CVL samples of women in the control group, including *L. crispatus* (the dominant species), *L*. *jensenii, L. gasseri, L. fermentum, L. salivarius,* and *L. vaginalis.* The lactobacillus specific profiles in the groups being studied, i.e., RM and INF, were narrower than in the controls, and *L. fermentum, L. salivarius,* and *L. vaginalis* were not found in any sample from RM and INF groups. Interestingly, *L. iners* was not isolated from any CVL sample of the control group, but was isolated from about one-third (5 out of a total of 18 lactobacillus-positive samples) of the samples of RM and INF groups. The levels of *L*. *salivarius* in vaginal swabs were also determined by means of species-specific qPCR. By means of using this technique, this bacterial species was detected only in the sample from one woman in the control group (7.29 log10 copies/swab). This woman was the only one identified as *L. salivarius-positive* (at a concentration of 7.3 log10 cfu/swab) using culture techniques. The *L. salivarius* strain isolated from a woman of this group was genetically different from *L. salivarius* CECT 5713 (results not shown).

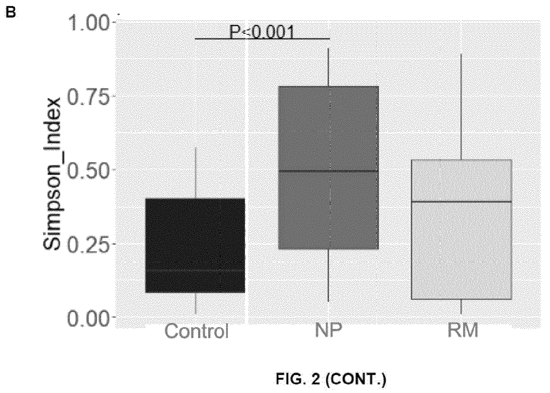

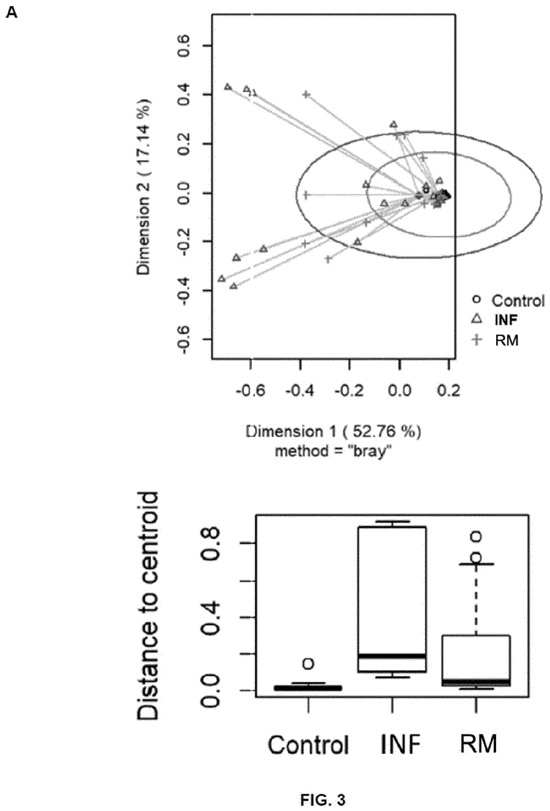

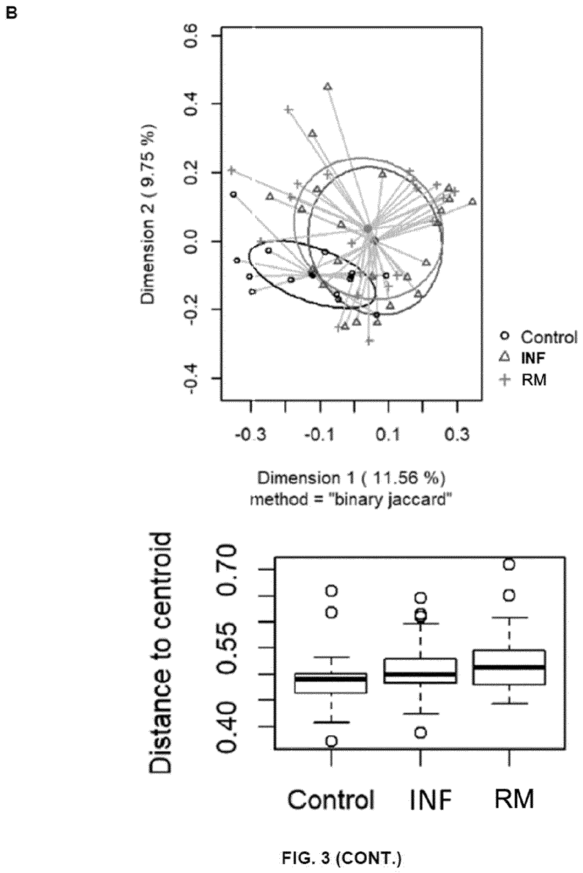

The sequencing analysis of the 16S rRNA gene of the CVL samples (n = 58) produced 4,363,364 high-quality filtered sequences, ranging from 33,160 to 139,044 per sample (median [IQR] = 73,383 [66,587-82,821]). Sequences were assigned to a total of 23 phyla and 453 genera. The alpha diversity analysis, calculated either by means of the Shannon index or the Simpson index, revealed significant differences between the vaginal microbiota of fertile women in the control group and women with infertility of unknown origin (p <0.001) (Figure 2). The beta diversity analysis, calculated according to the relative abundance of OTU (Bray-Curtis distance) and the presence/absence of OTU (Jaccard binary distance matrix), indicated that the vaginal bacterium of the 3 groups was clustered (p = 0.004 and p = 0.002, respectively) (Figure 3). Furthermore, the samples from fertile controls were more closely clustered (shorter distance to the centroid) according to the relative abundance of OTU (Bray-Curtis distance) than those from the RM and INF groups, which indicates that the bacterial profile in the CVL samples of the controls was highly uniform (Figure 3).

PCA analysis was performed for an initial evaluation of potentially dominant patterns in the bacteriological profile of the vaginal samples (Figure 4). The first two PCA dimensions explained 84.9% of the total variation present in the samples, with the first dimension (principal component) representing 74.7% and the second dimension representing 10.2% of the total variance. In general, this analysis revealed a greater similarity between the samples from the fertile control group and women with a history of repetitive miscarriage than between the fertile control group and women with infertility of unknown origin.

The comparison of the relative abundance (% of the total) of OTU at the phylum level in CVL samples of the three groups revealed statistically significant differences with respect to the 4 dominant phyla: Firmicutes, Actinobacteria, Proteobacteria, and Bacteroidetes (Table 3). Firmicutes was the most abundant and common (present in all the samples) phylum. The relative abundance of Firmicutes in the samples provided by fertile controls [median (IQR) = 95.01% (99.18% - 99.8%)] was greater than in the samples from women in the RM and INF groups [median (IQR) = 97.29% (72.34% - 99.35%) and 89.96% (52.46% -98.85%), respectively] (p <0.050 and p <0.001; Wilcoxon tests) (Table 3). In contrast, the median values (IQR) of the relative abundance of Actinobacteria, Proteobacteria, and Bacteroidetes were higher in women from RM and INF groups than in fertile controls (p <0.012, p <0.003, and p <0.006, respectively; Kruskal -Wallis rank tests) (Table 3).

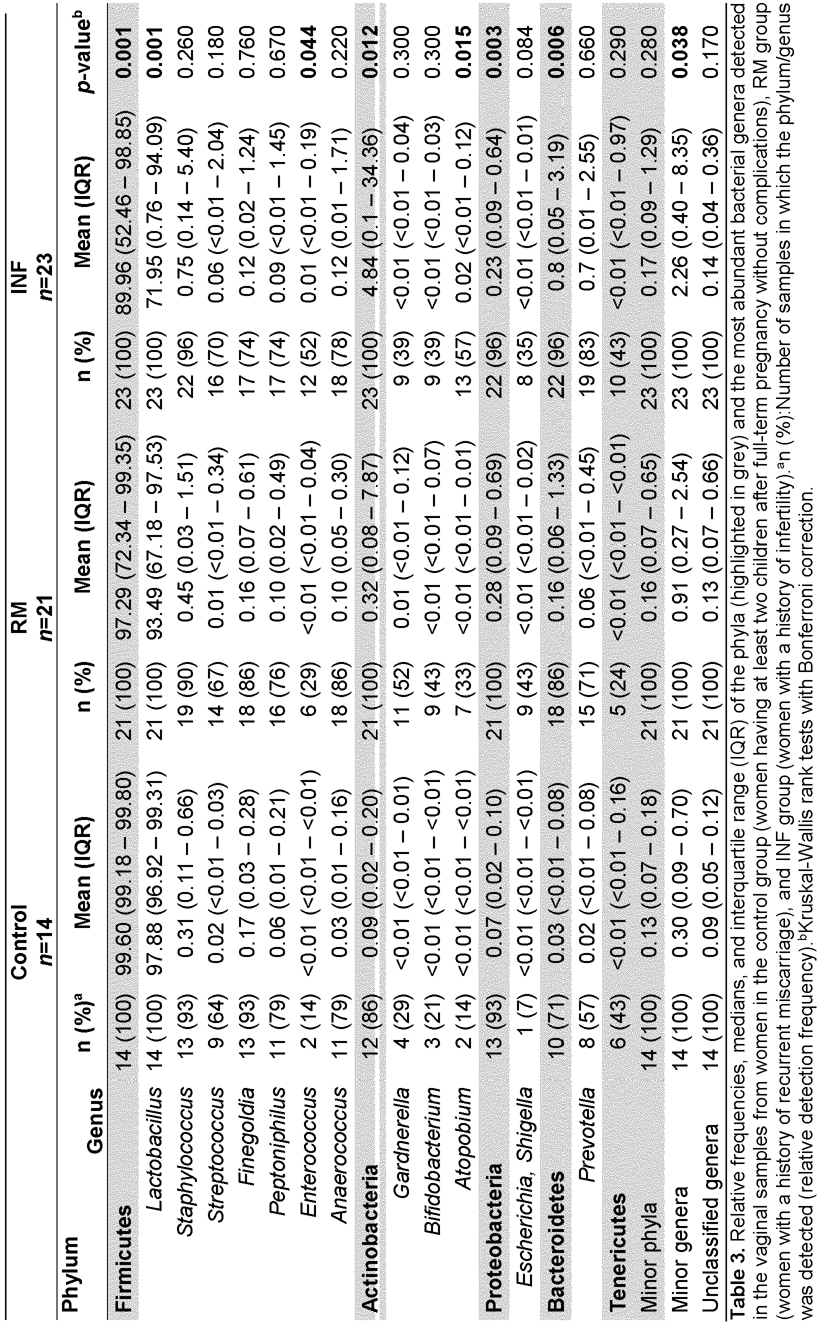

At the genus level, the only bacterial genus that was detected in all the samples was *Lactobacillus,* but there were significant differences in its relative abundance in the samples from the three groups (Table 3). The median (IQR) of the relative abundance of *Lactobacillus* in the samples from women in the RM and INF groups [93.49% (67.18%-97.53%) and 71.95% (0.76%-94.09%), respectively] was lower than in the samples from fertile control women [97.88% (96.92%-99.31%)] (p = 0.001; Kruskal-Wallis rank tests) (Table 3). Other genera were present in a variable number of samples, ranging between *96% (Staphylococcus* in the INF group) and 7% *(Escherichia*/*Shigella* in the control group), but the median of the relative abundance of any of these genera was less than <1%. At the genus level, the bacterial profile in some individual samples from women in the RM and INF groups did not differ from the bacterial profile of the samples from women in the fertile control group, which were extremely homogeneous. However, aberrant profiles with a reduced content or even the complete absence of *Lactobacillus* were recorded in some samples from women in the RM and INF groups (data not shown).

Generally, the comparison of the RM and INF groups at the start of the study revealed statistically significant differences between both groups (Figure 6). The vaginal pH was higher in women from the INF group than in women from the RM group, but the opposite was observed for TGFβ1 and VEGF. No differences were observed with respect to other characteristics, such as age, weight, height, Nugent score, TGFβ2, and viable lactobacillus counts (Figure 5).

### Main parameter of the clinical study: pregnancy and full-term pregnancy effectiveness (reproductive/pregnancy results)

In general, the administration of probiotics of the strain to women in the RM and INF groups led to 29 pregnancies out of 44 women participants. This means a pregnancy effectiveness of 66% with a Cl 95% of 51-78% (p = 0.017) (Table 4). Among these pregnancies, 25 were successful and 4 ended in miscarriage out of 44 women participants. This means a reproductive success effectiveness of 56% with a Cl 95% of 42-70% (p = 0.183) (Table 4). Interestingly, all the successful pregnancies led to full-term singleton pregnancies (gestational age ≥38 weeks). The gestational ages, the values of birth weight and length, and the sex of the babies are shown in Table 1.

**Table 4. Main results after probiotic treatment.**

| **Group** | **Pregnancy** | **Pregnancy effectiveness** | **Successful pregnancy** | **Reproductive success** |
|---|---|---|---|---|
| **Recurrent miscarriage (RM)** | 17/21 | 81% (47 - 29%) | 15/21 * | 71% (40 - 118%) |
| **Infertility (INF)** | 12/23 | 52% (27 - 91%) | 10/23* | 43% (21 - 80%) |
| **Difference between groups** | | 29% (-19 - 77%) | | 28% (-17 - 73%) |
| ***p*-value** | | 0.240 | | 0.219 |
| **Ratio (RM/INF)** | | 1.55 (0.70-3.56) | | 1.64 (0.69 - 4.09) |
| **Total** | 29/44 | 66% (51 - 78%) | 25/44 | 56% (42 - 70%) |

| | | | | |
|---|---|---|---|---|
| # Pearson chi-square ratio. *2 women in each group had a miscarriage. | | | | |

The women in the RM group had the highest rate of reproductive success since there were 17 pregnancies (15 full-term pregnancy and 2 miscarriages) out of 21 participants during the period of study. The rate in the INF group was lower but still notable: 12 pregnancies (10 full-term pregnancy and 2 miscarriages) out of 23 participants. The comparison of pregnancy effectiveness and the rates of successful pregnancy (CI 95%) between women in RM and INF [1.55 (0.70-3.56) and 1.64 (0.69-4.09, respectively) revealed that there were no statistically significant differences between both groups (Table 4). However, it should be pointed out that all the women in these groups had been subjected unsuccessfully to ART interventions in prior attempts to prevent a spontaneous miscarriage (RM group) or to become pregnant (INF group).

### Secondary response parameters associated with probiotic treatment: changes in the vaginal parameters in women in the RM group

There were no differences in age, weight, or height among those women in the RM group who ended up with a successful pregnancy (n = 15) and those who did not (n = 6).

However, differential changes were observed in this group of women (RM group) after probiotic treatment depending on the final pregnancy results (Table 5). In those women who were able to complete a successful pregnancy, the mean value (SD) of the vaginal pH was 1.13 (0.38) units lower if they became pregnant in comparison with those who did not. Similar changes were observed for the Nugent score which recorded 3.33 (1.11) units of difference between successful pregnancy and unsuccessful pregnancy (p = 0.000, one-way repeated-measures ANOVA) (Table 5, Figure 6).

**Table 5. Differences in characteristics and comparison of the changes in vaginal parameters among women who were able to complete full-term pregnancy (n = 15) and those who were not (n = 6) after intervention with L. salivarius CECT 5713 in the RM group which included women having histories of recurrent spontaneous miscarriage (n = 21).**

| | | **The intervention resulted in** | | |
|---|---|---|---|---|
| | | **pregnancy *n=*15** | **no pregnancy *n=*6** | ***p*-value#** |
| **pH** | | | | |
| | Initial | 5.58 (0.51) | 5.88 (0.45) | 0.221 |
| | Final | 4.45 (0.22) | 5.65 (0.48) | 0.000 |
| | Change | -1.13 (0.38) | -0.23 (0.30) | <0.001 |
| *p*-value† | | 0.000 | 0.002 | |

| **Nugent score** | | | | |
|---|---|---|---|---|
| | Initial | 5.87 (1.77) | 6.17 (1.17) | 0.708 |
| | Final | 2.53 (1.06) | 5.50 (1.05) | 0.000 |
| | Change | -3.33 (1.11) | -0.67 (0.82) | 0.000 |
| *p*-value† | | 0.000 | 0.102 | |

| **TGFβ1 (pg/mL)** | | | | |
|---|---|---|---|---|
| | Initial | 2.81 (0.54) | 2.15 (0.35) | 0.014 |
| | Final | 4.21 (0.29) | 2.47 (0.62) | 0.000 |
| | Change | 1.40 (0.62) | 0.32 (0.38) | <0.001 |
| *p*-value† | | 0.000 | 0.098 | |

| **TGFP2 (pg/mL)** | | | | |
|---|---|---|---|---|
| | Initial | 1.67 (0.26) | 1.15 (0.29) | <0.001 |
| | Final | 2.93 (0.34) | 1.30 (0.23) | 0.000 |
| | Change | 1.25 (0.34) | 0.15 (0.34) | 0.000 |
| *p*-value† | | 0.000 | 0.328 | |

| **VEGF (pg/mL)** | | | | |
|---|---|---|---|---|
| | Initial | 341 (124) | 109(11) | <0.001 |
| | Final | 743 (313) | 138(52) | <0.001 |
| | Change | 402 (252) | 29 (47) | 0.002 |
| *p*-value† | | 0.000 | 0.189 | |

| **Presence of lactobacilli [n (%)]** | | | | |
|---|---|---|---|---|
| | Initial | 9 (60) | 3 (50) | 0.523* |
| | Final | 15 (100) | 4 (67) | 0.071* |
| | Change | 6 (40) | 1 (17) | 0.613* |

| **Lactobacillus count (log cfu/swab)** | | | | |
|---|---|---|---|---|
| | Initial | 4.99 (0.99) | 5.20 (0.92) | 0.752 |
| | Final | 6.52 (0.60) | 4.74 (1.29) | <0.001 |
| | Change | 3.52 (2.99) | 0.56 (1.62) | 0.035 |

| **L. *salivarius* qPCR [n (%)]** | | | | |
|---|---|---|---|---|
| | Initial | nd | nd | - |
| | Final | 15 (100) | 3 (50) | 0.015* |

| ***L. salivarius* qPCR (copies/swab)** | | | | |
|---|---|---|---|---|
| | Initial | - | - | - |
| | Final | 6.85 (0.61) | 2.63 (1.17) | <0.000 |

| | | | | |
|---|---|---|---|---|
| # One-way ANOVA tests were used to evaluate the differences in the means between groups, except for the presence of lactobacilli.*Fisher's exact probability test for a 2 × 2 contingency table. † One-way ANOVA tests were used to determine if there were changes in each group of participants. | | | | |

The changes in concentrations of vaginal cytokines were also different in both groups after probiotic treatment. There were no modifications in vaginal concentrations of TGFβ1, TGFβ2, and VEGF in women who did not become pregnant, but there was a significant mean increase (SD) of 1.40 (0.62) pg/mL, 1.25 (0.34) pg/mL, and 402 (252) pg/mL], respectively, in those who did (Table 5, Figure 7). Furthermore, it should be taken into account that even before starting the treatment, there were already differences in the concentration of these cytokines among those who became pregnant and those who did not (Table 5, Figure 7).

Moreover, the probiotic treatment resulted in a mean increase (SD) in the lactobacillus counts of 3.52 (2.99) log10 cfu/ml in women who finally became pregnant, but not in those who did not (Table 5, Figure 6). The presence of *L. salivarius* [mean (SD) = 6.85 (0.61) copies/swab] was confirmed by means of qPCR in all the women who became pregnant, but only in 50% of the women with unsuccessful pregnancies, and then at a lower concentration [mean (SD) = 2.63 (1.17) copies/swab] (Table 5). The lactobacillus profile in the CVL samples obtained at the start of the probiotic treatment and after 6 months or until the diagnosis of pregnancy in Figure 8. The most significant differences were with respect to the presence of viable *L. salivarius* in most of the women (17/21) after probiotic treatment. Furthermore, *L. iners,* which was present in 3 women at the start of the study, was isolated at the end of the treatment from only 2 women who, in particular, did not terminate their pregnancy.

There were no differences in the metataxonomic profile at the genus level of the CVL samples from women in the RM group with respect to the pregnancy result (Table 6).

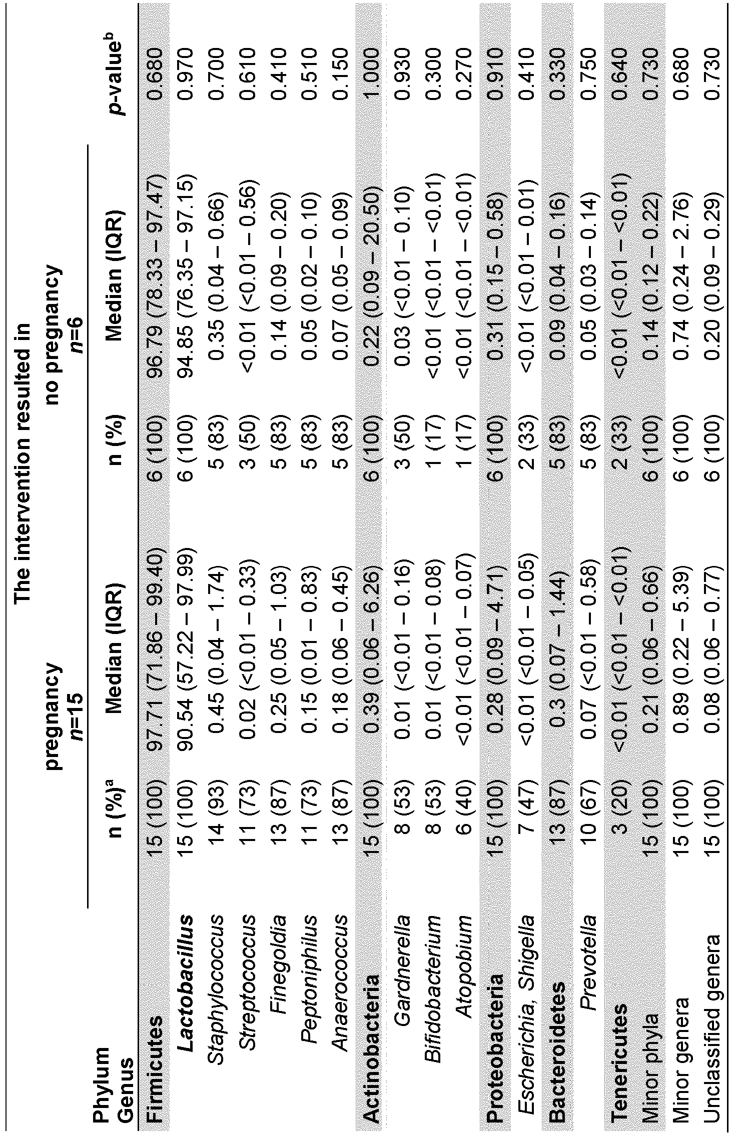

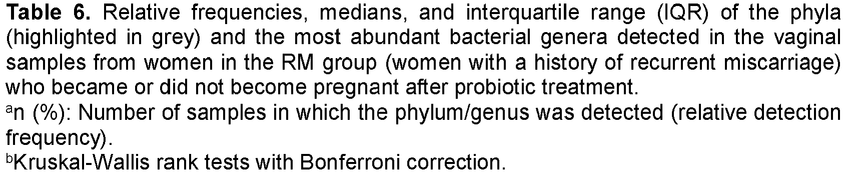

### Secondary response parameters associated with probiotic treatment: changes in vaginal parameters in women in the INF group

There were no differences in the age, weight, and height of the women in the INF group who became pregnant after probiotic treatment and those who did not (results not shown).

The CVL pH and Nugent score decreased significantly in all the women in the INF group after probiotic treatment (p <0.05; one-way repeated-measures ANOVA; Figure 9), although the magnitude of the change was smaller in women who did not become pregnant in comparison with those who did (Table 7, Figure 10). Specifically, the mean reductions (SD) in CVL pH and Nugent score in women who became pregnant were 1.32 (0.31) and 3.90 (0.74), respectively, and in women who did not become pregnant, these reductions were only 0.19 (0.18) and 0.54 (0.78), respectively. (Table 7, Figure 9).

**Table 7. Differences in characteristics and comparison of the changes in vaginal parameters among women who were able to complete full-term pregnancy (n = 10) and those who were not (n = 13) after intervention with L. salivarius CECT 5713 in the INF group which included women with a history of infertility of unknown origin (n=23).**

| | | **The intervention resulted in** | | |
|---|---|---|---|---|
| | | **pregnancy *n=*10** | **no pregnancy *n=*13** | ***p*-value#** |
| **pH** | | | | |
| | Initial | 5.85 (0.41) | 6.04 (0.26) | 0.190 |
| | Final | 4.53 (0.24) | 5.85 (0.25) | 0.000 |
| | Change | -1.32 (0.31) | -0.19 (0.18) | 0.000 |
| *p*-value† | | 0.000 | 0.002 | |

| **Nugent score** | | | | |
|---|---|---|---|---|
| | Initial | 6.00 (1.33) | 6.54 (1.27) | 0.334 |
| | Final | 2.10 (1.10) | 6.00 (1.00) | 0.000 |
| | Change | -3.90 (0.74) | -0.54 (0.78) | 0.000 |
| *p*-value† | | 0.000 | 0.028 | |

| **TGFβ1 (pg/mL)** | | | | |
|---|---|---|---|---|
| | Initial | 2.29 (0.44) | 2.11 (0.40) | 0.308 |
| | Final | 4.58 (0.42) | 2.18 (0.31) | 0.000 |
| | Change | 2.29 (0.34) | 0.08 (0.25) | 0.000 |
| *p*-value† | | 0.000 | 0.281 | |

| **TGFB2 (pg/mL)** | | | | |
|---|---|---|---|---|
| | Initial | 1.56 (0.26) | 1.16 (0.19) | <0.001 |
| | Final | 2.81 (0.30) | 1.26 (0.28) | 0.000 |
| | Change | 1.25 (0.24) | 0.10 (0.27) | 0.000 |
| *p*-value† | | 0.000 | 0.203 | |

| **VEGF (pg/mL)** | | | | |
|---|---|---|---|---|
| | Initial | 311 (101) | 82 (26) | 0.000 |
| | Final | 773 (253) | 87 (29) | 0.000 |
| | Change | 462 (166) | 6 (14) | 0.000 |
| | *p*-value† | 0.000 | 0.165 | |

| **Presence of lactobacilli [n (%)]** | | | | |
|---|---|---|---|---|
| | Initial | 3 (30) | 3 (19) | 0.537* |
| | Final | 10 (100) | 6 (46) | 0.007* |
| | Change | 7 (70) | 3 (19) | |

| **Lactobacillus count (log cfu/swab)** | | | | |
|---|---|---|---|---|
| | Initial | 5.00 (2.17) | 6.57 (0.51) | 0.290 |
| | Final | 6.46 (0.51) | 4.95 (1.66) | 0.017 |
| | Change | 3.05 (0.60) | 0.32 (0.77) | < 0.001 |

| ***L. salivarius* qPCR[n (%)]** | | | | |
|---|---|---|---|---|
| | Initial | nd | nd | - |
| | Final | 10 (100) | 4 (25) | 0.002* |

| ***L. salivarius* qPCR (copies/swab)** | | | | |
|---|---|---|---|---|
| | Initial | - | - | - |
| | Final | 6.48 (0.41) | 3.55 (0.39) | 0.000** |

| | | | | |
|---|---|---|---|---|
| #One-way ANOVA tests were used to evaluate the differences in the means between groups.*Fisher's exact probability test for a 2 × 2 contingency table. **One-way ANOVA; Kruskal-Wallis, p=0.007. | | | | |

The change in concentrations of vaginal cytokines after probiotic treatment was similar to that described in the RM group: there was no modification in vaginal concentrations of TGFβ1, TGFβ2, and VEGF in women who did not become pregnant, but there was a significant mean increase (SD) of 2.29 (0.34) pg/mL, 1.25 (0.24) pg/mL, and 462 (252) pg/mL], respectively, in women who did become pregnant (Table 7, Figure 9). In this group of women, even before starting treatment, the concentration of TGFβ2 and VEGF, but not TGFβ1, were already different among those women who became pregnant and those who did not (Table 7, Figure 9).

The probiotic treatment resulted in a higher number of CVL samples in which lactobacilli were detected (in all the women who finally became pregnant) and a higher mean bacterial count (SD) [(6.46 (0.51) log10 cfu/ml] than in those women who did not become pregnant (lactobacilli were detected in only 46% and the mean bacterial counts (SD) were 4.95 (1.66) log10 cfu/ml) (Table 7, Figures 9 and 10). Similarly, the presence of *L. salivarius* [mean (SD) = 6.85 (0.61) copies/swab] was confirmed by means of qPCR in all the women who became pregnant, but only in 25% of women with unsuccessful pregnancies, and then at a lower concentration [mean (SD) = 3.55 (0.39) copies/swab] (Table 7). The lactobacillus profile in the CVL samples obtained at the start of probiotic treatment and after 6 months or until the diagnosis of pregnancy in Figure 11 which shows that viable *L. salivarius* were detected in all the women who became pregnant, but only in 4 out of 13 of women who did not become pregnant after probiotic treatment.

There were no differences in the metataxonomic profile at the genus level of CVL samples from women in the RM group with respect to the pregnancy result (results not shown).

### Comparison of the vaginal parameters among all the women who became pregnant and women who did not in both RM and INF groups

The mean pH value in the CVL samples was a little more acidic in women who became pregnant [5.69 (0.48) units] than in those who did not [5.99 (0.33) units] (p = 0.024; one-way ANOVA) (Table 7). There were also differences in the concentration of vaginal cytokines at the start of the study according to the final pregnancy result, but the differences were similar to those already independently described for the RM and INF groups (Table 8; Figures 12 and 13). The only parameters that did not differ initially between both groups were the Nugent score, the detection frequency, and the lactobacillus count (Table 8). Overall, *Lactobacillus* was detected in all the women who became pregnant, but only in half of those who did not (p <0.001; Chi-square test). Furthermore, there was an increase of 1.6 log units in the lactobacillus counts after probiotic treatment in the group of women who became pregnant, but there were no changes in those who did not (Figure 14). The differences were also significant with respect to *L. salivarius* detection in CVL samples. This species was also detected at a high concentration [mean (SD) = 6.70 (0.56) copies/swab] in all the women who achieved successful pregnancy in comparison with those who did not (Figure 14).

**Table 8. Differences in characteristics and comparison of the changes in vaginal parameters among women who were able to complete full-term pregnancy (n = 25) and those who did not (n = 19) after intervention with L. salivarius CECT 5713, including all the women of RM and INF groups (n=44).**

| | | **The intervention resulted in** | | |
|---|---|---|---|---|
| | | **pregnancy** | **no pregnancy** | |
| | | ***n=*25** | | ***p*-value#** |
| | | | ***n=*19** | |
| **Age (years)** | | 39 (2.5) | 38.2 (2.7) | 0.302 |
| **Weight (kg)** | | 67.8 (8.0) | 66.7 (5.7) | 0.612 |
| **Height (cm)** | | 168 (7) | 167 (5) | 0.862 |

| **pH** | | | | |
|---|---|---|---|---|
| | Initial | 5.69 (0.48) | 5.99 (0.33 | 0.024 |
| | Final | 4.48 (0.23) | 5.78 (0.34 | 0.000 |
| | Change | -1.20 (0.36) | -0.21 (0.22 | 0.000 |
| *p*-value† | | 0.000 | 0.002 | |

| **Nugent score** | | | | |
|---|---|---|---|---|
| | Initial | 5.92 (1.58) | 6.42 (1.22) | 0.258 |
| | Final | 2.36 (1.08) | 5.84 (1.01) | 0.000 |
| | Change | -3.56 (1.00) | -0.58 (0.77) | 0.000 |
| *p*-value† | | 0.000 | 0.028 | |

| **TGFβ1 (pg/mL)** | | | | |
|---|---|---|---|---|
| | Initial | 2.60 (0.56) | 2.12 (0.37) | 0.002 |
| | Final | 4.36 (0.39) | 2.27 (0.43) | 0.000 |
| | Change | 2.76 (0.68) | 0.15 (0.31) | 0.000 |
| *p*-value† | | 0.000 | 0.281 | |

| **TGFβ2 (pg/mL)** | | | | |
|---|---|---|---|---|
| | Initial | 1.63 (0.26) | 1.16 (0.22) | 0.000 |
| | Final | 2.88 (0.32) | 1.27 (0.26) | 0.000 |
| | Change | 1.25 (0.30) | 0.12 (0.28) | 0.000 |
| | *p*-value† | 0.000 | 0.203 | |

| **VEGF (pg/mL)** | | | | |
|---|---|---|---|---|
| | Initial | 329 (114) | 90 (25) | 0.000 |
| | Final | 755 (285) | 103 (43) | 0.000 |
| | Change | 462 (219) | 13 (30) | 0.000 |
| *p*-value† | | 0.000 | 0.165 | |

| **Presence of lactobacilli [n (%)]** | | | | |
|---|---|---|---|---|
| | Initial | 12 (48) | 6 (32) | 0.273* |
| | Final | 25 (100) | 10 (46) | <0.001** |
| | Change | 7 (70) | 3 (19) | |

| **Lactobacillus count (log cfu/swab)** | | | | |
|---|---|---|---|---|
| | **Initial** | 4.99 (1.25) | 5.88 (1.00) | 0.150 |
| | Final | 6.47 (0.60) | 4.87 (1.45) | 0.000 |
| | Change | 4.07 (2.88) | 0.70 (1.64) | 0.000 |

| ***L. salivarius* qPCR[n (%)]** | | | | |
|---|---|---|---|---|
| | Initial | nd | nd | - |
| | Final | 25 (100) | 7 (37) | <0.001 |

| ***L. salivarius* qPCR (copies/swab)** | | | | |
|---|---|---|---|---|
| | Initial | - | - | - |
| | Final | 6.70 (0.56) | 3.16 (0.88) | 0.000** |

| | | | | |
|---|---|---|---|---|
| # One-way ANOVA tests were used to evaluate the differences in the means between groups. *Chi-square (Pearson) test for a 2 × 2 contingency table. ** Fisher's exact probability test for a 2 × 2 contingency table. ** Differences in the means of positive samples. | | | | |

### Comparison of the vaginal parameters among the control women, all the women who became pregnant, and women who did not in the RM and INF groups

The analysis of the vaginal parameters (pH, Nugent score, TGFβ1, TGFβ2, VEGF, lactobacillus count) revealed statistically significant differences among the three groups (control group, women in the RM and INF groups who experienced complete, full-term pregnancy, and women in the RM and INF groups who did not become pregnant or had a miscarriage) for all of said parameters (Table 9; Figure 15).

**Table 9. Comparison of vaginal parameters (pH, Nugent score, cytokines, and lactobacillus count) between the participants in the control group (n=14) and the participants in the RM and INF groups (jointly) who achieved pregnancy (n = 25) or who did not (n = 19) after probiotic treatment.**

| | | **Control *n=*14** | **RM+INF Pregnancy *n=*25** | **RM+INF No pregnancy *n=*19** | ***p-*value#** |
|---|---|---|---|---|---|
| **pH** | | | | | |
| | Mean | 4.53a | 4.48a | 5.78b | 0.000 |
| | SD | 0.25 | 0.23 | 0.36 | |
| | CI 95% | (4.38-4.68) | (4.39-4.58) | (5.62-5.95) | |
| | [min-max] | [4.20-5.00] | [4.20-4.90] | [5.10-6.40] | |

| **Nugent score** | | | | | |
|---|---|---|---|---|---|
| | Mean | 1.79a | 2.36b | 5.84b | 0.000 |
| | SD | 0.98 | 1.08 | 1.01 | |
| | CI 95% | (1.27-2.30) | (1.92-2.80) | (5.35-6.33) | |
| | [min-max] | [0.00-4.00] | [1.00-4.00] | [4.00-7.00] | |

| **TGFβ1 (pg/mL)** | | | | | |
|---|---|---|---|---|---|
| | Mean | 4.83a | 4.36b | 2.27c | 0.000 |
| | SD | 0.36 | 0.39 | 0.43 | |
| | CI 95% | (4.65-5.01) | (4.20-4.52) | (2.06-2.48) | |
| | [min-max] | [4.20-5.30] | [3.80-5.20] | [1.70-3.60] | |

| **TGFβ2 (pg/mL)** | | | | | |
|---|---|---|---|---|---|
| | Mean | 3.22a | 2.88b | 1.27c | 0.000 |
| | SD | 0.27 | 0.32 | 0.26 | |
| | CI 95% | (3.07-3.38) | (2.75-3.01) | (1.15-1.40) | |
| | [min-max] | [2.70-3.70] | [2.30-3.60] | [0.90-1.70] | |

| **VEGF (pg/mL)** | | | | | |
|---|---|---|---|---|---|
| | Mean | 406.0a | 755.0b | 103.0c | 0.016 |
| | SD | 379.0 | 285.0 | 43.2 | |
| | CI 95% | (322.0-490.0) | (206.0-343.0) | (116.0-247.0) | |
| | [min-max] | [187.0-625.0] | [637.0-872.0] | [82.4-124.0] | |

| **Lactobacilli** | | | | | |
|---|---|---|---|---|---|
| | Positive women [n, (%)] | 14 (100) | 25 (100) | 10 (53) | <0.001* |
| | Count (log₁₀cfu/swab) | | | | |
| | Mean** | 7.24**a** | 6.47**b** | 4.87**c** | 0.000 |
| | SD | 0.30 | 0.60 | 1.45 | |
| | CI 95% | (6.89-7.60) | (6.22-6.72) | (3.83-5.90) | |
| | [min-max] | [6.80-7.70] | [5.15-7.40] | [3.00-7.10] | |

| | | | | | |
|---|---|---|---|---|---|
| SD: standard deviation; Cl: confidence interval, TGFβ1, transforming growth factor β1; TGFβ2, transforming growth factor β2; VEGF, vascular endothelial growth factor. # One-way ANOVA tests were used to evaluate the differences in the means between groups. The different letters shown in bold in the same row indicate statistically significant differences between groups. *Freeman-Halton extension of Fisher exact probability tests for a 2 × 3 contingency table. **Mean in those women in whom lactobacilli could be isolated from their samples. | | | | | |

### Antimicrobial activity of L. salivarius CECT 5713 against vaginal and cervical pathogens

*L. salivarius* CECT 5713 showed antimicrobial inhibitory activity (zone of inhibition > 2 mm around the line) against all the indicator organisms used in this study, both under aerobic conditions and under anaerobic conditions. To explain the compound/compounds responsible for the antimicrobial activity, this non-bacteriogenic strain was selected for hydrogen peroxide, lactate, and/or acetate production. A quantitative assay showed that the mean amount (± SD) of hydrogen peroxide produced by this strain was 0.729 µg/ml (± 0.122) under aerobic conditions. This compound was not produced when the assays were performed under anaerobic conditions. Mean concentrations (± SD) of L-lactic acid in the supernatants obtained from MRS cultures (initial pH = 6.2) of the strain were 11.07 ± 0.42 mg/ml and 11.69 ± 0.58 mg/ml under aerobic and anaerobic conditions, respectively, whereas the mean pH values in the supernatants were 3.91 and 3.86 under aerobic and anaerobic conditions, respectively. D-Lactate was not detected in the culture supernatants. Mean concentrations (± SD) of acetic acid in the same supernatants were 0.70 mg/ml (± 0.11) and 0.46 mg/ml (± 0.14) under aerobic and anaerobic conditions, respectively.

**Table 10. Antimicrobial activity and congregation of L. salivarius CECT 5713 with respect to various cervicovaginal pathogens.**

| Strain | Halo of inhibition (mm) | Co-aggregation |
|---|---|---|
| *G. vaginalis* MP14 | 4.6 | ++ |
| *G. vaginalis* MP17 | 4.0 | ++ |
| *G. vaginalis* MP20 | 4.4 | ++ |
| *G. vaginalis* MP24 | 4.3 | ++ |
| *G. vaginalis* MP29 | 4.0 | ++ |
| *S. agalactiae* MP07 | 2.5 | ++ |
| *S. agalactiae* MP12 | 2.2 | ++ |
| *S. agalactiae* MP46 | 2.1 | ++ |
| *C. albicans* MP09 | 3.5 | ++ |
| C. *albicans* MP18 | 3.4 | ++ |
| *C. albicans* MP31 | 2.9 | ++ |
| *C. glabrata* MP33 | 3.0 | + |
| *C. glabrata* MP37 | 2.4 | + |
| *C. parapsilosis* MP36 | 3.1 | + |
| *C. parapsilosis* MP48 | 2.6 | + |
| *U. urealyticum* MP39 | 4.0 | + |
| *U. urealyticum* MP57 | 3.5 | + |

| | | |
|---|---|---|
| ^{a}Co-aggregation is defined as visible clusters of bacteria and are classified as (++): large and dense clusters; (+): small and sparse clusters; (-): no visible clusters, following the criterion of Younes *et al.,* (2012). Complete reference: Younes, J. A., van der Mei, H. C., van den Heuvel, E., Busscher, H. J., and Reid. G. (2012). Adhesion forces and co-aggregation between vaginal staphylococci and lactobacilli. PLoS One, 7, e36917. doi: 10.1371/journal.pone.0036917 | | |

### Co-aggregation of L. salivarius CECT 5713 with vaginal pathogens and adhesion to vaginal cells

The strain was able to form large and well-defined co-aggregates with all the selected vaginal and cervical pathogens. The co-aggregation was particularly intense with strains of *G*. *vaginalis,* S. *agalactiae,* and C. *albicans.* In this study, the tested strain strongly adhered to the vaginal epithelial cells, with a mean of 329 ± 46 adherent lactobacilli in 20 random microscopic fields. The mean value (± SD) for *L. salivarius* CECT 9145, a control strain with high adhesion to vaginal cells, was 336 ± 52 adherent lactobacilli.

### A-amylase activity of L. salivarius CECT 5713

The extracellular amylase production by *L. salivarius* CECT 5713 was observed by means of the zone of clearance around the colonies (~ 2.0 mm) when treated with an iodine solution. Later, when the α-amylase activity was measured, this strain showed a high α-amylase activity level (0.83 U/ml) after 16 h (concentration of *L. salivarius* CECT 5713: ~ 8.6 log10 cfu/ml), and it could be detected in supernatants at a similar level for 48 h (when the assay ended).

## Claims

1. A strain of the species Lactobacillus salivarius deposited in the Spanish Type Culture Collection (CECT) with access number 5713 for use in the treatment and/or prevention of infertility or recurrent pregnancy loss in a female subject.

2. The strain for use according to claim 1, wherein the infertility is unexplained infertility.

3. The strain for use according to claim 1, wherein the recurrent pregnancy loss is unexplained recurrent pregnancy loss.

4. The strain for use according to any of claims 1 to 3, wherein the strain is administered to a woman with infertility or recurrent pregnancy loss in combination with assisted reproductive treatment.

5. The strain for use according to any of claims 1 to 4, wherein the strain is administered orally.

6. The strain for use according to any of claims 1 to 5, wherein the strain is administered for a period of time of at least 3 months or until diagnosis of pregnancy.

7. The strain for use according to claim 6, wherein the strain is administered for a period of time of at least 6 months.

8. The strain for use according to any of claims 1 to 7, wherein if pregnancy is diagnosed, the administration of the strain is maintained at least until the 15th week of pregnancy.

9. The strain for use according to any of claims 1 to 8, wherein the strain is administered at a daily dose comprised of 8.5 log 10 cfu and 9.5 log cfu.

10. The strain for use according to claim 9, wherein the strain is administered at a daily dose of approximately 9 log 10 cfu.

11. An *in vitro* method to monitor the effect of a treatment for infertility or recurrent pregnancy loss in a female subject with a strain of the species Lactobacillus salivarius deposited in the CECT under accession number 5713 comprising:
i. determining the level in a sample of the mucosa of the vagina and/or of the mucosa of the cervix of said female subject of a factor of growth selected from the group consisting of VEGF, TGF-β1 and TGF-β2 and
ii. compare said level with a reference value obtained from the same subject before therapy,
where an increase in said level with respect to the reference value is indicative that the treatment is being effective and a decrease or lack of change in said level with respect to the reference value is indicative that the treatment is being ineffective.

## Patentansprüche

1. Stamm der Spezies Lactobacillus salivarius, der in der spanischen Kulturensammlung (CECT) mit der Zugangsnummer 5713 hinterlegt ist, zur Verwendung bei der Behandlung und/oder Vorbeugung von Unfruchtbarkeit oder wiederkehrendem Schwangerschaftsverlust bei einer Probandin.

2. Stamm zur Verwendung nach Anspruch 1, wobei die Unfruchtbarkeit eine ungeklärte Unfruchtbarkeit ist.

3. Stamm zur Verwendung nach Anspruch 1, wobei der wiederkehrende Schwangerschaftsverlust ein ungeklärter wiederkehrender Schwangerschaftsverlust ist.

4. Stamm zur Verwendung nach einem der Ansprüche 1 bis 3, wobei der Stamm einer Frau mit Unfruchtbarkeit oder wiederkehrendem Schwangerschaftsverlust in Kombination mit einer assistierten Reproduktionsbehandlung verabreicht wird.

5. Stamm zur Verwendung nach einem der Ansprüche 1 bis 4, wobei der Stamm oral verabreicht wird.

6. Stamm zur Verwendung nach einem der Ansprüche 1 bis 5, wobei der Stamm für einen Zeitraum von mindestens 3 Monaten oder bis zur Diagnose einer Schwangerschaft verabreicht wird.

7. Stamm zur Verwendung nach Anspruch 6, wobei der Stamm für einen Zeitraum von mindestens 6 Monaten verabreicht wird.

8. Stamm zur Verwendung nach einem der Ansprüche 1 bis 7, wobei, wenn eine Schwangerschaft diagnostiziert wird, die Verabreichung des Stamms mindestens bis zur 15. Schwangerschaftswoche aufrechterhalten wird.

9. Stamm zur Verwendung nach einem der Ansprüche 1 bis 8, wobei der Stamm in einer Tagesdosis verabreicht wird, die 8,5 log 10 KBE und 9,5 log KBE umfasst.

10. Stamm zur Verwendung nach Anspruch 9, wobei der Stamm in einer Tagesdosis von etwa 9 log 10 KBE verabreicht wird.

11. *In vitro* -Verfahren zur Überwachung der Wirkung einer Behandlung für Unfruchtbarkeit oder wiederkehrenden Schwangerschaftsverlust bei einer Probandin mit einem Stamm der Spezies Lactobacillus salivarius, der beim CECT unter der Zugangsnummer 5713 hinterlegt ist, umfassend:
i. Bestimmen des Spiegels eines Wachstumsfaktors, ausgewählt aus der Gruppe bestehend aus VEGF, TGF-β1 und TGF-β2, in einer Probe der Schleimhaut der Vagina und/oder der Schleimhaut des Gebärmutterhalses der Probandin und
ii. Vergleichen dieses Spiegels mit einem Referenzwert, der von derselben Probandin vor der Therapie erhalten wurde,
wobei eine Erhöhung des Spiegels in Bezug auf den Referenzwert darauf hinweist, dass die Behandlung wirksam ist, und eine Abnahme oder fehlende Änderung des Spiegels in Bezug auf den Referenzwert darauf hinweist, dass die Behandlung unwirksam ist.

## Revendications

1. Souche de l'espèce Lactobacillus salivarius déposée dans la collection espagnole de cultures de type (CECT) sous le numéro d'accès 5713 pour une utilisation dans le traitement et/ou la prévention de l'infertilité ou de la perte de grossesse récurrente chez un sujet féminin.

2. Souche à utiliser selon la revendication 1, dans laquelle l'infertilité est une infertilité inexpliquée.

3. Souche à utiliser selon la revendication 1, dans laquelle la perte de grossesse récurrente est une perte de grossesse récurrente inexpliquée.

4. Souche utilisée selon l'une quelconque des revendications 1 à 3, dans laquelle la souche est administrée à une femme souffrant d'infertilité ou de pertes de grossesse récurrentes, en association avec un traitement de procréation assistée.

5. Souche à utiliser selon l'une quelconque des revendications 1 à 4, dans laquelle la souche est administrée par voie orale.

6. Souche à utiliser selon l'une quelconque des revendications 1 à 5, dans laquelle la souche est administrée pendant une période d'au moins 3 mois ou jusqu'au diagnostic de grossesse.

7. Souche à utiliser selon la revendication 6, dans laquelle la souche est administrée pendant une période d'au moins 6 mois.

8. Souche à utiliser selon l'une quelconque des revendications 1 à 7, dans laquelle, si une grossesse est diagnostiquée, l'administration de la souche est maintenue au moins jusqu'à la 15e semaine de grossesse.

9. Souche à utiliser selon l'une quelconque des revendications 1 à 8, dans laquelle la souche est administrée à une dose quotidienne comprise entre 8,5 log 10 cfu et 9,5 log cfu.

10. Souche à utiliser selon la revendication 9, dans laquelle la souche est administrée à une dose quotidienne d'environ 9 log 10 cfu.

11. Procédé *in vitro* pour contrôler l'effet d'un traitement de l'infertilité ou d'une perte de grossesse récurrente chez un sujet féminin avec une souche de l'espèce Lactobacillus salivarius déposée dans la CECT sous le numéro d'accession 5713 consistant à :
i. déterminer le niveau, dans un échantillon de la muqueuse du vagin et/ou de la muqueuse du col de l'utérus dudit sujet féminin, d'un facteur de croissance choisi dans le groupe constitué par le VEGF, le TGF-β1 et le TGF-β2 et
ii. comparer ledit niveau à une valeur de référence obtenue chez le même sujet avant le traitement,
où une augmentation dudit niveau par rapport à la valeur de référence indique que le traitement est efficace et une diminution ou l'absence de changement dudit niveau par rapport à la valeur de référence indique que le traitement est inefficace.
